(19)

Europäisches Patentamt
European Patent Office
Office européen des brevets

(11)  **EP 3 311 858 B1**

(12)  **EUROPEAN PATENT SPECIFICATION**

(45) Date of publication and mention
of the grant of the patent:
**07.08.2019  Bulletin 2019/32**

(21) Application number: **16811771.1**

(22) Date of filing: **17.06.2016**

(51) Int Cl.:
*A61M 1/02* *(2006.01)*     *B01D 39/16* *(2006.01)*

(86) International application number:
**PCT/JP2016/068190**

(87) International publication number:
**WO 2016/204297 (22.12.2016 Gazette 2016/51)**

(54) **BLOOD-PROCESSING FILTER AND BLOOD-PROCESSING METHOD**

BLUTAUFBEREITUNGSFILTER UND BLUTAUFBEREITUNGSVERFAHREN

FILTRE DE TRAITEMENT DU SANG, ET PROCÉDÉ DE TRAITEMENT DU SANG

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.06.2015   JP 2015122447
17.06.2015   JP 2015122450**

(43) Date of publication of application:
**25.04.2018   Bulletin 2018/17**

(73) Proprietor: **Asahi Kasei Medical Co., Ltd.
Tokyo 100-0006 (JP)**

(72) Inventor: **SHIMADA, Nobukazu
Tokyo 101-8101 (JP)**

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

(56) References cited:
**EP-A1- 2 450 431      WO-A1-2015/088019
JP-A- 2002 204 910    JP-A- 2006 014 887
JP-A- 2007 050 013**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Technical Field

The present invention relates to a blood processing filter that is used for removing unnecessary components from blood, i.e., whole blood and blood products (liquids obtained by preparation from whole blood, and these liquids supplemented with various carbonized materials), and a blood processing method using this blood processing filter. Also disclosed is a filter element for use in this blood processing filter.

Background Art

In the field of blood transfusion, so-called blood component transfusion of separating a blood component necessary for a recipient from a whole blood product and transfusing the blood component has generally been practiced in addition to so-called whole blood transfusion of transfusing a whole blood product in which blood collected from a donor is supplemented with an anticoagulant. The blood component transfusion includes red cell transfusion, platelet transfusion, plasma transfusion, and the like depending on the type of the blood component necessary for a recipient, and the blood product used for these transfusions includes a red cell product, a platelet product, a plasma product, and the like.

Furthermore, so-called leukocyte-free blood transfusion of transfusing a blood product after removing leukocytes contained in the blood product has become widespread recently. This is because it has been revealed that relatively slight adverse reactions accompanying blood transfusion, such as headache, nausea, chill, or febrile non-hemolytic reaction, and severe adverse reactions having serious effects on a recipient, such as alloantigen sensitization, viral infection, or post-transfusion GVHD, are mainly caused by leukocytes contained in the blood product used in blood transfusion. For preventing relatively slight adverse reactions such as headache, nausea, chill, or fever, it is considered necessary to remove leukocytes in the blood product until the residual rate becomes from $10^{-1}$ to $10^{-2}$ or less. Also, for preventing alloantigen sensitization or viral infection, which is a severe adverse reaction, it is considered necessary to remove leukocytes until the residual rate becomes from $10^{-4}$ to $10^{-6}$ or less.

Furthermore, in recent years, leukocyte removal therapy by the extracorporeal circulation of blood has been practiced in the treatment of diseases such as rheumatism or ulcerative colitis, and high clinical effects have been obtained.

Currently, methods of removing leukocytes from the blood product are roughly classified into two types: a centrifugation method of separating and removing leukocytes by using a centrifuge and utilizing the difference in specific gravity among blood components, and a filter method of removing leukocytes by using a filter material consisting of a fiber assembly such as a nonwoven fabric or a porous structure having continuous pores, or the like. The filter method which removes leukocytes by adhesion or adsorption is most widely used at present because of having the advantages that the operation is simple and the cost is low, for example.

In recent years, new demands for leukocyte removal filters have been proposed in the medical practice. One of the demands is to improve the recovery rate of useful components used as the blood product, such as plasma proteins. Although blood, which is a raw material for the blood product, is valuable blood that is covered by blood donation with good intentions in most cases, there is a problem that plasma proteins and red cell products that have been adsorbed on a filter material in a leukocyte removal filter and thus become impossible to recover are disposed of together with the filter and end up in the garbage. Therefore, it is of significant importance to reduce the amount of the useful components adsorbed as compared with the current leukocyte removal filter and improve the recovery rate.

Thus, a leukocyte removal filter apparatus packed with a smaller amount of a filter material than ever by using a leukocyte removal filter material whose leukocyte removal performance per unit volume is high has been desired for satisfying the aforementioned demands in the medical practice. It is expected that the amount of blood remaining in the filter is decreased with decrease in the packing amount of the filter material so that the recovery rate of useful components can be improved over the conventional filter apparatus.

In the market, there has been a demand for the leukocyte removal filter to process a desired amount of blood in a short time. Therefore, the leukocyte removal filter apparatus is thought to have a shape in which the cross section is equal to or larger than that of the conventional apparatus and the thickness of the filter material is thinner. However, for decreasing the thickness of the filter material while maintaining the leukocyte removal performance, it is necessary to enhance the leukocyte removal performance per unit volume.

Meanwhile, the mechanism of leukocyte removal with a filter material such as a fiber assembly or a porous structure having continuous pores is considered to be based mainly on the adhesion or adsorption of leukocytes contacted with the filter material surface onto the filter material surface. Accordingly, in order to satisfy the aforementioned demands, studies to decrease the fiber diameter of the nonwoven fabric or increase the bulk density, for example, have been conducted as an approach for improvement in the leukocyte removal performance of the conventional filter material (see Patent Literatures 1 and 2).

Furthermore, a leukocyte removal method that attains high leukocyte removal performance and has a short processing time without causing clogging by using a leukocyte removal filter in which a specific structure in the thickness direction, i.e., the flow direction of liquids, is rendered uniform over the entire filtration surface of the nonwoven fabric has been proposed as another approach (see Patent Literature 3).

EP 2 450 431 discloses nonwoven fabrics used in blood separation apparatuses preferably including at least one of polyester fibers, polypropylene fibers, nylon fibers, and acrylic fibers. Preferred examples of polyester include polyethylene terephthalate and polybutylene terephthalate because they enhance the red blood cell separation efficiency and the white blood cell separation efficiency. The nonwoven fabric more preferably includes nylon, polypropylene, and/or polybutylene terephthalate in order to enhance the red blood cell separation efficiency and the white blood cell separation efficiency.

Citation List

Patent Literature

Patent Literature 1: Japanese Patent Laid-Open No. 60-193468
Patent Literature 2: U.S. Patent No. 5580465
Patent Literature 3: Japanese Patent No. 4134043
Patent Literature 4: Japanese Patent No. 5710244

Summary of Invention

Technical Problem

However, leukocyte removal performance may not be improved in some cases even when the physical properties of filter elements are optimized according to the description of Patent Literatures 1 to 3.

Moreover, even use of filter elements prepared according to the description of Patent Literature 1 to 3 does not improve the resistance of filters to centrifugation because they are not meant to increase the mechanical properties of the filter elements.

Blood processing filters are generally prepared by allowing a rigid container or a flexible container to hold a filter element.

In this respect, if the filter element is not correctly held by the container, blood is leaked to the outside from the filter element-holding part during blood filtration. As a result, leukocyte removal performance is presumably reduced.

Patent Literature 1 to 3 focus only on the control of the physical properties of filter elements themselves and do not discuss the holding state of filter elements in filters.

In light of the problems of the conventional techniques, an object of the present invention is to provide a blood processing filter with a filter element stably held in the filter.

Solution to Problem

The present inventors have conducted diligent studies on blood processing filters having a supported filter element containing a nonwoven fabric, and consequently found that a filter element can be stably held in a filter by setting the rebound strength per unit basis weight of the filter element to a predetermined level or larger.

The present inventors have further revealed that the resistance to centrifugation of a blood processing filter having a filter element integrally joined with a flexible container as disclosed in Figure 7 of Patent Literature 4 is improved by setting the rebound strength per unit basis weight of a nonwoven fabric contained in the filter element to a predetermined level or larger.

Advantageous Effects of Invention

The filter of the present invention according to claim 1 or 2 enables the filter element to be stably held in a filter even when any of rigid containers and flexible containers are used as a container. This can improve the filtration performance (leukocyte removal performance, etc.) of the filter.

In the case of preparing a filter by assembling a flexible container with a filter element such that the filter element is sandwiched in the flexible container, the filter element of the present invention is also effective for improving the resistance of the filter to centrifugation.

Brief Description of Drawings

Figure 1 is a schematic view of a blood processing filter equipped with a blood processing filter element according to one example of the embodiment of the present invention.

Figure 2 is a cross-sectional view of a blood processing filter equipped with a blood processing filter element according to one example of the embodiment of the present invention.

Figure 3 is a schematic view of a blood processing filter equipped with a blood processing filter element according to another example of the embodiment of the present invention.

Figure 4 is a cross-sectional view of a blood processing filter equipped with a blood processing filter element according to another example of the embodiment of the present invention.

Description of Embodiments

Hereinafter, modes for carrying out the present invention according to claim 1 or 2 (hereinafter, referred to as the present embodiment) will be described in detail.

In the present embodiment, examples of the nonwoven polymer fabric (hereinafter also referred to as "nonwoven fabric") include,

but are not particularly limited to, resin fibers formed by spinning a resin such as polyethylene terephthalate (PET) or polybutylene terephthalate (PBT).

The filter element for a blood processing filter of the present embodiment comprises a nonwoven fabric, wherein the rebound strength per unit basis weight before steam heat treatment is 0.3 ($N·m^2/g$) or larger, and the shape of the filter element is preferably a sheet shape.

In this context, the steam heat treatment refers to exposure to steam of 100°C or higher.

The blood processing filter of the present embodiment is used for removing unfavorable components from blood or a liquid containing a blood component and suitably used, particularly, for removing leukocytes from a leukocyte-containing liquid.

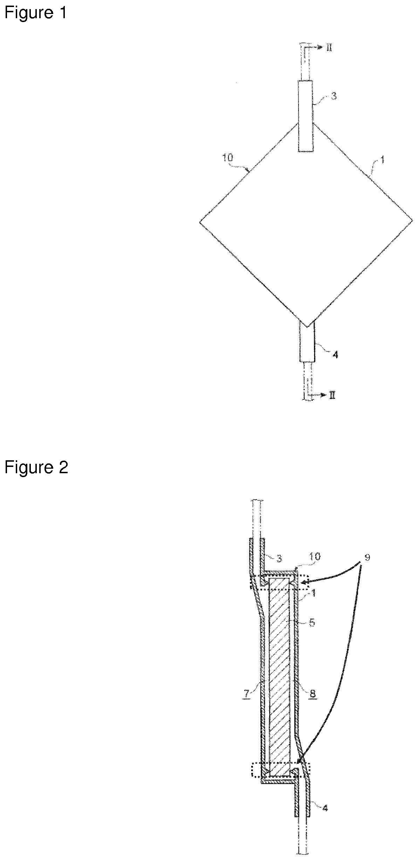

The filter element for blood processing of the present embodiment is housed in a container of a blood processing filter and used for removing unnecessary components such as leukocytes from blood. Figure 1 is a schematic view of one example of the blood processing filter of the present embodiment, and Figure 2 is a cross-sectional view taken along the II-II line in the blood processing filter of Figure 1.

A blood processing filter 10 shown in Figures 1 and 2 has, for example, a flat rigid container 1 and a filter element 5 housed in the inside thereof. The container 1 which houses the filter element 5 is constituted by at least an inlet-side container member having a first port 3 and an outlet-side container member having a second port 4. The space within the flat container 1 is partitioned by the filter element 5 into space 7 on the first port side and space 8 on the second port side.

The filter 10 of the present embodiment assumes a structure where the inlet-side container member and the outlet-side container member are disposed to sandwich the filter element 5, and these two container members hold the filter element 5 such that, for example, holding parts ,which are respectively provided on the members, bind outer edges 9 of the filter element 5. In this respect, the filter element 5 exhibits rebound force in a direction against the bonding pressure from the container members (holding parts, etc.). It has been revealed that insufficiency of this rebound force reduces the filtration performance of the blood processing filter 1 in such a way that a phenomenon in which blood sneaks through the gaps between the filter element and the container members (holding parts, etc.) and directly runs into the outlet space from the inlet space without passing through the filter element (side leak phenomenon) occurs, or in an extreme case, bonding pressure does not properly act, and thus blood is leaked to the outside from the filter.

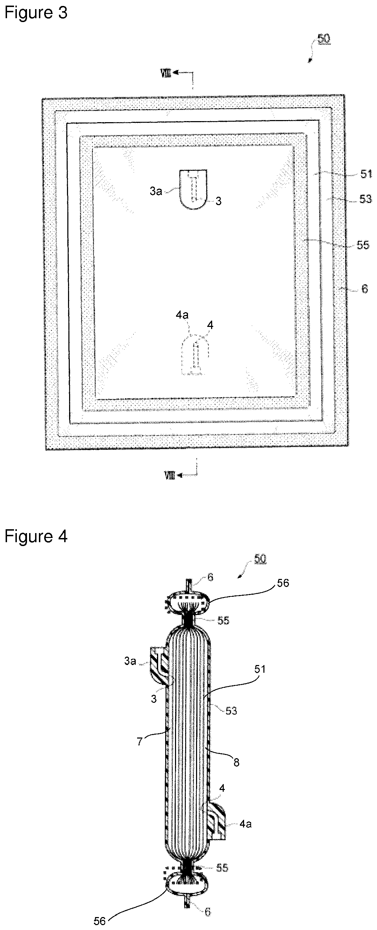

The blood processing filter of the present embodiment may be prepared using a flexible container. Figure 3 shows a schematic view of a blood processing filter in this form. Figure 4 is a cross-sectional view taken along the VII-VII line in Figure 3.

As shown in Figures 3 and 4, a blood processing filter 50 has a flat flexible container 53 and a filter element 51 housed therein. The container 53 which houses the filter element 51 has a first port 3 disposed at the end part of one principal surface and a second port 4 disposed at the end part of the other principal surface. The space within the flat container 1 is partitioned by the blood processing filter element 5 into space 7 on the first port side and space 8 on the second port side.

The filter 50 is further equipped with bonded parts 55 at which the filter element 51 and the flexible container are bonded by welding or the like, and protruding nonwoven fabric parts 56 on the outer side thereof. A general blood processing filter comprising a flexible container may be subjected to centrifugal operation together with blood at the time of blood product preparation before blood filtration. It has been revealed that if the rebound strength of the filter element is not sufficient, strong centrifugal force is applied to the bonded parts 55 during this operation due to a lack of the cushioning properties of the protruding nonwoven fabric parts 56, consequently causing cracks or peeling in the bonded parts 55. Such cracks in the bonded parts 55 facilitate leaking blood to the outside of the filter or to the protruding nonwoven fabric side from the bonded parts during blood filtration. This may consequently lead to hazards such as reduction in leukocyte removal performance, product contamination, and reduction in recovery amount.

In addition, a lack of the cushioning properties of the protruding nonwoven fabric parts 56 might cause cracks upon application of some force to the bonded parts 55 at the time of filter handling other than centrifugal treatment.

In the present embodiment, a filter element having a rebound strength per unit basis weight of a predetermined level or higher is used. Thus, for a blood processing filter in which a filter element is held by being sandwiched with a rigid container, a side leak phenomenon during blood filtration is suppressed. For a blood processing filter in which a filter element is held by being bonded with a flexible container, the cushioning properties of the protruding nonwoven fabric parts are enhanced to thereby reduce load on the bonded parts of the container to the filter element during centrifugal treatment or the like, consequently suppressing defects such as cracks. This achieves improvement in filtration performance and/or improvement in handleability.

In the case of carrying out a blood processing method which involves subjecting a blood processing filter together with a blood bag to centrifugal operation, a blood processing filter comprising the filter element of the present embodiment housed in a flexible container can be suitably used for preparing a blood product such as a red cell product from a whole blood product or the like by the centrifugal operation. Before the centrifugal operation, the filter having a flexible container is loaded in a centrifugal cup together with the blood bag. In this respect, the filter is easily handled during the loading because the strength of the bonded parts is increased as compared with conventional filters. Thus, the working time required for the loading is drastically reduced as compared with conventional products. In addition, defects such as cracks caused by load on the bonded parts are also suppressed during centrifugal operation, and the risk of wasting products can be reduced compared with conventional products.

In the present embodiment, the rebound strength per unit basis weight of the filter element 5 or 51 before steam heat treatment is 0.3 $(N\cdot m^2/g)$ or larger, preferably 0.32 $(N\cdot m^2/g)$ or larger, more preferably 0.35 $(N\cdot m^2/g)$ or larger. The rebound strength per unit basis weight according to the present invention is determined by dividing the rebound strength of the filter element compressed into a thickness of 4.4 mm by the basis weight (mass (g) per $m^2$) $(g/m^2)$ of the filter element and can be measured with an autograph tester.

The measurement method will be described in the steps 1) to 3) given below. For example, an autograph tester (Autograph AG-10KNI) from Shimadzu Corp. can be used as a compression apparatus.

1) The filter element contained in the blood processing filter is cut into 7.35 cm square in the planar direction, and the mass (g) of the filter element thus cut (sample) is measured. This mass is divided by the area (54 $cm^2$) to calculate the basis weight $(g/m^2)$ of the filter element.

2) The central part of the sample obtained in the step 1) is compressed at a compression rate of 5 mm/min using a cylindrical compression jig having a diameter of 3.3 cm. The rebound strength (N) of the sample compressed into a thickness of 4.4 mm is measured.

3) From the basis weight and the rebound strength of the filter element measured in the steps 1) and 2), the rebound strength per unit basis weight $(g/m^2)$ ([rebound strength (N) measured in the step 2)] / [basis weight $(g/m^2)$ measured in the step 1)]) is calculated.

In the present embodiment, the rebound strength per unit basis weight of the filter element before steam heat treatment is 0.3 $(N\cdot m^2/g)$ or larger. The rebound strength per unit basis weight is improved by steam heat treatment for the purpose of, for example, sterilizing the filter element. For example, the rebound strength measured under the measurement conditions of the step 2) and calculated by the method of the step 3) is known to be improved by approximately 10% by sterilizing the sample prepared in the step 1) by steam heating under conditions of 115°C and 240 minutes.

This is probably because the crystallinity of the nonwoven fabric contained in the filter element is increased by the steam heat treatment. Another possible reason is that fibers constituting the nonwoven fabric are enlaced by the fusion between the fibers, and this enlacement improves the mechanical strength of the nonwoven fabric.

Thus, in the case of using the filter element of the present embodiment after steam heat treatment for the purpose of sterilizing the filter element, its rebound strength per unit basis weight is a value much larger than 0.3 $(N\cdot m^2/g)$ in use.

The filter element having a rebound strength of 0.3 $(N\cdot m^2/g)$ or larger per unit basis weight before steam heat treatment can be easily produced, for example, by using a nonwoven fabric having a high rebound strength per unit basis weight before steam heat treatment as the nonwoven fabric to be contained therein.

The WC value of the nonwoven fabric contained in the filter element for a blood processing filter, corresponding to a basis weight of 40 g/m$^2$ is preferably 2.0 (gf·cm/cm$^2$) or smaller, more preferably 1.7 (gf·cm/cm$^2$) or smaller, further preferably 1.4 (gf·cm/cm$^2$) or smaller. If the WC value is larger than 2.0 (gf·cm/cm$^2$), the nonwoven fabric is easily compressed to thereby cause a side leak phenomenon (in the case of using a rigid container) during blood filtration or cracks (in the case of using a flexible container) or the like during centrifugation, consequently leading to reduction in leukocyte removal performance or filter handleability. In particular, for a filter using a flexible container, when the WC value of the nonwoven fabric contained in the filter element, corresponding to a basis weight of 40 g/m$^2$ is 2.0 (gf·cm/cm$^2$) or smaller, this is preferred because the strength of the bonded parts of the container to the filter element is improved, and resistance to centrifugation is also improved. This effect of improving the strength of the bonded parts of the container to the filter element by setting the WC value of the nonwoven fabric corresponding to a basis weight of 40 g/m$^2$ to 2.0 (gf·cm/cm$^2$) or smaller is obtained irrespective of the value of the rebound strength of the filter element before steam heat treatment.

In this context, the WC value (gf·cm/cm$^2$) of the nonwoven fabric according to the present embodiment refers to the work of compression measured using KES (Kawabata Evaluation System) and serves as an index for the evaluation of the texture properties of nonwoven fabrics. The measurement method using KES is described in "The Standardization and Analysis of Texture Evaluation (second edition)", Sueo Kawabata, issued by Texture Evaluation and Standardization Committee, the Textile Machinery Society of Japan (July 10, 1980).

Specifically, the measurement method can be performed by the following procedures using a compression tester (e.g., Compression Tester KES-G5 manufactured by Kato Tech Co., Ltd.).

The nonwoven fabric is cut into a size of 5 cm × 20 cm, and the nonwoven fabric piece is mounted on a sample table. The nonwoven fabric piece is compressed using a round pressure plate having an area of 2 cm$^2$ until the maximum compressive load becomes 500 gf/cm$^2$. The compression rate is set to 0.05 mm/sec. The process of restoration is also assayed at the same rate as above. The work of compression (WC) is represented by the expression given below. In the expression, Tm, To, and P represent the thickness of the nonwoven fabric under a load of 500 gf/cm$^2$, the thickness of the nonwoven fabric under a load of zero, and the load (gf) at the time of measurement, respectively. The WC values of 3 different sites in the nonwoven fabric piece are measured, and an average value thereof is used as the WC value of the nonwoven fabric.

$$WC = \int_{T_0}^{T_m} PdT$$

The WC value depends largely on the basis weight of the nonwoven fabric. Therefore, the WC value corresponding to a basis weight of 40 g/m$^2$ can be calculated by the following method.

First, 3 nonwoven fabrics having a basis weight of 40 g/m$^2$ or smaller are provided, and their respective basis weights and WC values are measured. Next, two out of the 3 nonwoven fabrics thus assayed are stacked such that the basis weight is 40 g/m$^2$ or larger. The WC value of the two nonwoven fabrics in a stacked state is measured. After the completion of WC value measurement as to a total of 3 combinations, a linear regression equation of the basis weight and the WC value is determined. The WC value corresponding to a basis weight of 40 g/m$^2$ can be determined from the equation.

The basis weight of two nonwoven fabrics may fall short of 40 g/m$^2$. In this case, a plurality of nonwoven fabrics are stacked such that the basis weight of the stacked nonwoven fabrics is 40 g/m$^2$ or larger, followed by WC value measurement. Next, the WC value of a fewer number of nonwoven fabrics can be measured such that the basis weight of the stacked nonwoven fabrics is 40 g/m$^2$ or smaller. The WC value is measured for all combinations of the nonwoven fabrics in which the basis weight of the stacked nonwoven fabrics is 40 g/m$^2$ or smaller. A linear regression equation of the basis weight and the WC value is determined. The WC value corresponding to a basis weight of 40 g/m$^2$ can be determined from the equation.

The filter element may further comprise a nonwoven fabric having a nonionic hydrophilic group and a basic nitrogen-containing functional group in a surface portion. For example, the fiber itself constituting the nonwoven fabric may have the nonionic hydrophilic group and the basic nitrogen-containing functional group in its surface portion, or a coat layer formed on the nonwoven fabric may have the nonionic hydrophilic group and the basic nitrogen-containing functional group in its surface portion.

The surface portion of the nonwoven fabric refers to the surface portion of the coat layer when the surface of the nonwoven fabric is coated with a coat layer containing a monomer and/or a polymer, etc., and refers to the surface portion of spun fibers when no coat layer is formed on the fibers.

The filter element comprising the nonwoven fabric having a nonionic hydrophilic group and a basic nitrogen-containing functional group in a surface portion can enhance the affinity of the nonwoven fabric for leukocytes in blood while enhancing the blood product permeability of the nonwoven fabric. Thus, leukocyte removal can be efficiently performed.

When the filter element comprises two or more nonwoven fabrics (mentioned later), at least one of the nonwoven fabrics can have the nonionic hydrophilic group and the basic nitrogen-containing functional group in the surface portion.

The ratio of the basic nitrogen-containing functional group to the total of the nonionic hydrophilic group and the basic nitrogen-containing functional group in the surface portion is preferably from 0.2 to 4.0% by mass, more preferably from 0.3 to 1.5% by mass. The ratio of the basic nitrogen-containing functional group can be measured by analysis based on NMR, IR, TOF-SIMS, or the like. The ratio between the basic nitrogen-containing functional group and the nonionic hydrophilic group can be set as described above to thereby secure stable wettability for blood and also efficiently perform leukocyte removal while suppressing the unnecessary clogging of blood components such as platelets.

Examples of the nonionic hydrophilic group include alkyl groups, alkoxy group, carbonyl groups, aldehyde groups, phenyl groups, amide groups, and hydroxyl groups. Examples of the basic nitrogen-containing functional group include amino groups represented by $-NH_2$, $-NHR_1$, $-NR_2R_3$, or $-N^+R^4R^5R^6$ ($R^1$, $R^2$, $R^3$, $R^4$, $R^5$, and $R^6$ each represent an alkyl group having from 1 to 3 carbon atoms).

The coat layer can contain, for example, a copolymer having a monomer unit having the nonionic hydrophilic group and a monomer unit having the basic nitrogen-containing functional group. Examples of the monomer unit having the nonionic hydrophilic group include units derived from 2-hydroxyethyl (meth)acrylate, 2-hydroxypropyl (meth)acrylate, vinyl alcohol, (meth)acrylamide, N-vinylpyrrolidone, and the like. Among these monomers, 2-hydroxyethyl (meth)acrylate is preferably used in view of easy availability, easy handling during polymerization, performance when blood flows, etc. The monomer unit of vinyl alcohol is usually formed by hydrolysis after polymerization of vinyl acetate.

Examples of the monomer unit having the basic nitrogen-containing functional group include units derived from: derivatives of (meth)acrylic acid such as diethylaminoethyl (meth)acrylate, dimethylaminoethyl (meth)acrylate, dimethylaminopropyl (meth)acrylate, and 3-dimethylamino-2-hydroxypropyl (meth)acrylate; styrene derivatives such as p-dimethylaminomethylstyrene and p-diethylaminoethylstyrene; vinyl derivatives of nitrogen-containing aromatic compounds such as 2-vinylpyridine, 4-vinylpyridine, and 4-vinylimidazole; derivatives in which the vinyl compounds described above are converted to quaternary ammonium salts with alkyl halides or the like; and the like. Among these monomers, diethylaminoethyl (meth)acrylate and dimethylaminoethyl (meth)acrylate are preferably used in view of easy availability, easy handling during polymerization, performance when blood flows, etc.

The mass of the coat layer is, for example, from approximately 1.0 to 40.0 mg with respect to 1 g in total of the masses of the nonwoven fabric and the coat layer.

The mass of the coat layer can be calculated by, for example, the following procedures: the nonwoven fabric before carrying the coat layer is dried for 1 hour in a dryer set to 60°C, and then left for 1 hour or longer in a desiccator, followed by the measurement of the mass (A g). The nonwoven fabric having the coat layer is similarly dried for 1 hour in a dryer of 60°C and then left for 1 hour or longer in a desiccator, followed by the measurement of the mass (B g). The amount of the coat layer is calculated according to the following expression:

$$\text{Mass (mg/g) of the coat layer with respect to 1 g in total of the nonwoven fabric and the coat layer} = (B - A) \times 1000 / B.$$

The coat layer containing the polymer (copolymer) can be formed by, for example, a method which involves dipping the nonwoven fabric in a polymer solution containing the polymer and a solvent, and then removing the solvent from the polymer solution attached to the nonwoven fabric.

The nonwoven fabric contained in the filter element 5 of the present embodiment preferably has a formation index of 15 or larger and 70 or smaller corresponding to a thickness of 0.3 mm. If the formation index is larger than 70, the structure in the thickness direction of the nonwoven fabric is non-uniform relative to the filtration surface direction so that blood does not flow evenly in the nonwoven fabric. Therefore, leukocyte removal performance is reduced. On the other hand, if the formation index is smaller than 15, clogging is more likely to occur due to a rise in liquid-flow resistance so that processing speed is slowed down. The formation index is more preferably 15 or larger and 65 or smaller, further preferably 15 or larger and 60 or smaller, particularly preferably 15 or larger and 50 or smaller, most preferably 15 or larger and 40 or smaller.

The formation index in the present embodiment is a value obtained by irradiating the nonwoven fabric with light from underneath, detecting the transmitted light with a charge-coupled device camera (hereinafter, abbreviated to a "CCD camera"), and multiplying the coefficient of variation (%) of the absorbance of the porous body (nonwoven fabric) detected by each pixel of the CCD camera by ten.

In the present embodiment, the formation index can be measured with, for example, a formation tester FMT-MIII (Nomura Shoji Co., Ltd.; manufactured in 2002; S/N: 130). The basic setting of the tester is not changed after shipment from the factory, and the measurement can be carried out such that the total number of pixels of a CCD camera is, for example, approximately 3400. Specifically, the measurement can be performed by adjusting the measurement size to 7 cm × 3 cm (one pixel size = 0.78 mm × 0.78 mm) such that the total number of pixels is approximately 3400. Alternatively, the measurement size may be changed according to the shape of a sample such that the total number of pixels is equal to 3400.

The formation index depends largely on the thickness of the nonwoven fabric. Therefore, the formation index corresponding to a thickness of 0.3 mm is calculated by the following method.

First, 3 nonwoven fabrics having a thickness of 0.3 mm or smaller are provided, and their respective formation indexes and thicknesses are measured. The thicknesses at arbitrary four points are measured at a measurement pressure of 0.4 N using a constant-pressure thickness meter (e.g., Ozaki Mfg. Co., Ltd., model FFA-12), and an average value thereof is used as the thickness of the nonwoven fabric. Next, two out of the 3 nonwoven fabrics thus assayed are stacked such that the thickness is 0.3 mm or larger. The formation index and the thickness of the two nonwoven fabrics in a stacked state are measured. After the completion of formation index measurement as to a total of 3 combinations, a linear regression equation of the thickness and the formation index is determined. The formation index corresponding to a thickness of 0.3 mm is determined from the equation.

The thickness of two nonwoven fabrics may fall short of 0.3 mm. In this case, a plurality of nonwoven fabrics are prepared and stacked such that the thickness of the stacked nonwoven fabrics is 0.3 mm or larger, followed by formation index measurement. Next, the number of the stacked nonwoven fabrics is reduced such that the thickness of the stacked nonwoven fabrics becomes 0.3 mm or smaller, and the formation index is measured. The formation index is measured for all combinations of the nonwoven fabrics in which the thickness of the stacked nonwoven fabrics is 0.3 mm or smaller. A linear regression equation of the thickness and the formation index is determined. The formation index corresponding to a thickness of 0.3 mm can be determined from the equation.

The 3 or more nonwoven fabrics used in the formation index measurement are cut out from a single filter element, and they are usually nonwoven fabrics having substantially the same quality, i.e., nonwoven fabrics having the same physical properties (material, fiber diameter, bulk density, etc.). However, if the required number of nonwoven fabrics having substantially the same quality for the measurement cannot be obtained from a single filter element, the measurement can be performed by using nonwoven fabrics from filter elements of the same type in combination therewith.

The specific method for calculating the formation index is also described in the paragraphs [0016] to [0018] of Patent Literature 3.

The specific surface area of the nonwoven fabric contained in the filter element 5 of the present embodiment is preferably 0.8 m$^2$/g or larger and 3.2 m$^2$/g or smaller. If the specific surface area is larger than 3.2 m$^2$/g, there is a tendency that useful components such as plasma proteins are adsorbed onto the filter element during blood processing so that the recovery rate of the useful components is reduced. If the specific surface area is smaller than 0.8 m$^2$/g, there is a tendency that leukocyte removal performance is reduced as compared with conventional filter elements because the amount of leukocytes adsorbed is decreased.

The specific surface area of the nonwoven fabric is more preferably 1.0 m$^2$/g or larger and 3.2 m$^2$/g or smaller, further preferably 1.1 m$^2$/g or larger and 2.9 m$^2$/g or smaller, particularly preferably 1.2 m$^2$/g or larger and 2.9 m$^2$/g or smaller, most preferably 1.2 m$^2$/g or larger and 2.6 m$^2$/g or smaller.

The specific surface area according to the present embodiment refers to the surface area of the nonwoven fabric per unit mass and is a value measured by a BET adsorption method using nitrogen as an adsorption gas. The specific surface area can be measured using, for example, Tristar 3000 apparatus manufactured by Micromeritics Japan.

A larger specific surface area of the nonwoven fabric means that there is larger area which can adsorb cells and plasma proteins, etc. during blood processing using a filter element containing the nonwoven fabric in a predetermined mass.

The airflow resistance of the nonwoven fabric contained in the filter element 5 of the present embodiment is preferably 25 Pa·s·m/g or larger and 100 Pa·s·m/g or smaller, more preferably 30 Pa·s·m/g or larger and 90 Pa·s·m/g or smaller, further preferably 40 Pa·s·m/g or larger and 80 Pa·s·m/g or smaller.

If the airflow resistance is smaller than 25 Pa·s·m/g, there is a tendency that the number of contacts with leukocytes is decreased so that the leukocytes are difficult to capture. If the airflow resistance of the nonwoven fabric is larger than 100 Pa·s·m/g, there is a tendency that clogging by blood cells is increased so that processing speed is decreased.

The airflow resistance of the nonwoven fabric of the present embodiment is a value measured as differential pressure generated when air flows at a predetermined flow rate in the nonwoven fabric, and is a value obtained by placing the nonwoven fabric on a vent hole of an air permeability testing apparatus (e.g., manufactured by Kato Tech Co., Ltd., KES-F8-AP1), measuring a pressure drop (Pa·s/m) generated when air is allowed to flow for approximately 10 seconds, and further dividing the obtained pressure drop by the basis weight (g/m$^2$) of the nonwoven fabric. In this respect, the measurement is performed for five samples cut out from different sites, and an average value thereof is used as the airflow resistance.

Higher airflow resistance of the nonwoven fabric means that air is less likely to penetrate the nonwoven fabric, and the fibers constituting the nonwoven fabric are entangled in a dense or uniform state, and indicates that the nonwoven fabric has the property of hindering a blood product from flowing. On the other hand, lower airflow resistance of the nonwoven fabric means that the fibers constituting the nonwoven fabric are entangled in a coarse or non-uniform state, and indicates that the nonwoven fabric has the property of facilitating the flow of a blood product.

The nonwoven fabric contained in the filter element 5 of the present embodiment preferably has a mean flow pore size of smaller than 8.0 $\mu$m. If the mean flow pore size is larger than 8.0 $\mu$m, there is a tendency that the number of contacts with leukocytes is decreased so that the leukocytes are difficult to capture. If the mean flow pore size is smaller than 1.0 $\mu$m, there is a tendency that clogging by blood cells is increased so that processing speed is decreased. The mean flow pore size is more preferably 1.5 $\mu$m or larger and 7.5 $\mu$m smaller, further preferably 2.5 $\mu$m or larger and 7.0 $\mu$m or smaller, particularly preferably 3.5 $\mu$m or larger and 6.5 $\mu$m or smaller, most preferably 4.5 $\mu$m or larger and 6.5 $\mu$m or smaller.

The mean flow pore size of the nonwoven fabric of the present embodiment can be measured in accordance with ASTM F316-86 using Perm Porometer CFP-1200AEXS (automatic pore size distribution measurement system for porous materials) manufactured by Porous Materials, Inc. (PMI). A nonwoven fabric having a larger mean flow pore size facilitates the flow of a blood product, but reduces leukocyte removal performance. On the other hand, a nonwoven fabric having a smaller mean flow pore size improves leukocyte removal performance, but hinders a blood product from flowing and is also more likely to be clogged.

The filter element of the present embodiment may be constituted by one nonwoven fabric or may be constituted by a plurality of nonwoven fabrics. The filter element constituted by a plurality of nonwoven fabrics may be constituted by nonwoven fabrics of a single type or may be constituted by nonwoven fabrics of plural types.

When the filter element is constituted by nonwoven fabrics of plural types, it is preferred that the filter element should have a first nonwoven fabric layer which is disposed upstream and removes microaggregates, and a second nonwoven fabric layer which is disposed downstream of the first nonwoven fabric layer in order to remove leukocytes and the like. Each of the first and second nonwoven fabric layers may be one nonwoven fabric or may consist of a plurality of nonwoven fabrics. Each of the first and second nonwoven fabric layers each consisting of a plurality of nonwoven fabrics may be constituted by nonwoven fabrics of a single type or may be constituted by nonwoven fabrics of plural types.

The first nonwoven fabric layer disposed on the inlet side is preferably a nonwoven fabric layer consisting of a nonwoven fabric having an average fiber diameter of from 3 to 60 $\mu$m, from the viewpoint of aggregate removal. The second nonwoven fabric layer is preferably a nonwoven fabric layer consisting of a nonwoven fabric having an average fiber diameter of from 0.3 to 3.0 $\mu$m from the viewpoint of leukocyte removal.

A post-filter layer may be further disposed, if necessary, downstream of the second nonwoven fabric layer.

The number of nonwoven fabrics constituting each nonwoven fabric layer can be appropriately selected in consideration of removal performance for leukocytes and the like required for the blood processing filter, a processing time, or balance thereof, etc., and may be, for example, one sheet for each.

In this form, the first nonwoven fabric layer of the filter element is disposed upstream (on the inlet side) of the second nonwoven fabric layer, and the nonwoven fabric constituting the second nonwoven fabric layer has a smaller average fiber diameter than that of the nonwoven fabric constituting the first nonwoven fabric layer. Even if aggregates are formed in blood, the loose nonwoven fabric of the upstream (inlet-side) first nonwoven fabric layer thereby captures the aggregates to decrease the number of aggregates arriving at the fine nonwoven fabric of the downstream second nonwoven fabric layer. Thus, the clogging of the filter element by aggregates is suppressed. Particularly, the nonwoven fabric constituting the first nonwoven fabric layer has an average fiber diameter of from 3 to 60 $\mu$m and is therefore effective for suppressing the clogging of the filter element. Also, the nonwoven fabric of the second nonwoven fabric layer has an average fiber diameter of smaller than 3 $\mu$m and can therefore prevent reduction in leukocyte removal performance.

The average fiber diameter of the nonwoven fabric constituting the first nonwoven fabric layer is more preferably from 4 to 40 $\mu$m, further preferably from 30 to 40 $\mu$m and/or from 10 to 20 $\mu$m, because the clogging of the filter element can be suppressed more reliably. The average fiber diameter of the nonwoven fabric constituting the second nonwoven fabric layer is preferably 0.3 $\mu$m or larger because clogging by leukocytes and the like is prevented. The average fiber diameter is further preferably from 0.5 to 2.5 $\mu$m, particularly, from the viewpoint of leukocyte removal performance, etc.

A third nonwoven fabric layer consisting of a nonwoven fabric having an average fiber diameter of from 1.2 to 1.5 $\mu$m and/or from 0.9 to 1.2 $\mu$m may be further disposed for use downstream of the second nonwoven fabric layer.

The first nonwoven fabric layer containing a nonwoven fabric having a thick average fiber diameter and the second nonwoven fabric layer containing a nonwoven fabric having a thin average fiber diameter may be alternately arranged. In this case, it is preferred that they are arranged in alternating order of the first nonwoven fabric layer, the second nonwoven fabric layer, the first nonwoven fabric layer, the second nonwoven fabric layer ... from the inlet side, from the viewpoint of improvement in flowability by cascade structure formation.

In the present embodiment, each nonwoven fabric contained in the filter element is preferably constituted by fibers having a substantially single fiber diameter.

When fibers having a thinner fiber diameter are added to fibers having a substantially single fiber diameter to produce a nonwoven fabric, the resulting nonwoven fabric has a decreased pore size and facilitates clogging by blood. On the other hand, when fibers having a thicker fiber diameter are added to fibers having a substantially single fiber diameter to produce a nonwoven fabric, the resulting nonwoven fabric has a decreased specific surface area and facilitates reduction in leukocyte removal performance.

The average fiber diameter according to the present embodiment refers to a value determined according to the following procedures: Several points from a portion found to be substantially uniform of the nonwoven fabric actually constituting the filter element or one or more nonwoven fabrics having substantially the same quality thereof are selected as samples. Photographs showing images of diameters of fibers in the nonwoven fabric samples are taken using a scanning electron microscope.

The photographs are continuously taken until the diameters of 100 fibers in total are photographed. The diameters of all the fibers appearing in the photographs thus obtained are measured. In this context, the diameter refers to the width of the fiber in the direction perpendicular to the fiber axis. A value obtained by dividing the sum of all the measured fiber diameters by the number of fibers is used as the average fiber diameter. Here, when a plurality of fibers are overlapped so that the diameter of a fiber hidden behind another fiber cannot be measured, when a plurality of fibers are melted, for example, to form a thick fiber, when fibers significantly differing in diameter coexist, or when the boundary of the fibers is not clear due to the incorrect focus of a photograph, such data is not counted.

In the case where the filter element contains a plurality of nonwoven fabrics, if the measured average fiber diameter for each nonwoven fabric is evidently different, this means that these nonwoven fabrics are of different types. In this case, their average fiber diameters are separately measured again by finding the interface between the different nonwoven fabrics. In this context, the phrase "average fiber diameter is evidently different" refers to the case where a significant difference is statistically observed.

For a blood processing filter having a plate-like and flexible container, particularly, a post-filter layer is preferably disposed downstream of the second nonwoven fabric layer, because the flow of blood is prevented from being inhibited in such a way that filter element is pressed against the outlet-side container due to positive pressure on the inlet side generated during filtration and further, the outlet-side container is tightly contacted with the filter element due to negative pressure on the outlet side, and also because the weldability between the flexible container and the filter element is enhanced.

As the post-filter layer, a filtration medium known in the art, such as a fibrous porous medium (e.g., nonwoven fabrics, woven fabrics, and meshes), or a porous body having three-dimensional network continuous pores, can be employed. Examples of materials for these filtration media include polypropylene, polyethylene, styrene-isobutylene-styrene copolymers, polyurethane, and polyester. A post-filter layer made of a nonwoven fabric is preferred from the viewpoint of productivity and the welding strength of the blood processing filter. A post-filter layer having a plurality of protrusions by embossing or the like is particularly preferred because the flow of blood is rendered more uniform.

The surface of each nonwoven fabric constituting the filter element may be modified by a technique known in the art, such as coating, chemical treatment, or radiation treatment, for the purpose of controlling selective separation properties for blood cells, surface hydrophilicity, etc.

For more reliably suppressing the clogging of the filter element, the bulk density of the nonwoven fabric constituting the first nonwoven fabric layer is preferably from 0.05 to 0.50 $g/cm^3$ and may be more preferably from 0.10 to 0.40 $g/cm^3$. If the bulk density of the nonwoven fabric of the first nonwoven fabric layer exceeds 0.50 $g/cm^3$, the nonwoven fabric might be clogged by the capture of aggregates or leukocytes, resulting in a reduced filtration rate. On the other hand, if the bulk density falls below 0.05 $g/cm^3$, aggregate capture performance might be reduced so that the nonwoven fabric of the second nonwoven fabric layer is clogged, resulting in a reduced filtration rate. In addition, the mechanical strength of the nonwoven fabric may be reduced.

The "bulk density of the nonwoven fabric" is determined by cutting out samples with a size of 2.5 cm $\times$ 2.5 cm from the nonwoven fabric at a site thought to be homogeneous, measuring the basis weight ($g/m^2$) and the thickness (cm) of the samples by methods mentioned later, and dividing the basis weight by the thickness. In this respect, the measurement of the basis weight and the thickness is performed for three samples cut out from different sites, and an average value thereof is used as the bulk density.

The basis weight of the nonwoven fabric is determined with a sample having a size of 2.5 cm $\times$ 2.5 cm cut out from the nonwoven fabric at a site thought to be homogeneous, measuring the weight of the samples, and converting this weight to a mass per unit square meter. Also, the thickness of the nonwoven fabric is determined with a sample having a size of 2.5 cm $\times$ 2.5 cm cut out from the nonwoven fabric at a site thought to be homogeneous, and measuring the thickness of its central part (one site) by a constant-pressure thickness meter. The load pressure of the constant-pressure thickness meter is set to 0.4 N, and the area of the measurement part is set to 2 $cm^2$.

The bulk density of the nonwoven fabric constituting the second nonwoven fabric layer is preferably from 0.05 to 0.50 $g/cm^3$, more preferably from 0.07 to 0.40 $g/cm^3$, further preferably from 0.10 to 0.30 $g/cm^3$. If the bulk density of the nonwoven fabric of the second nonwoven fabric layer is larger than 0.50 g/cm$^3$, there is a tendency that the flow resistance of the nonwoven fabric is increased, and clogging by blood cells is accordingly increased so that processing speed is decreased. On the other hand, if the bulk density is smaller than 0.05 g/cm$^3$, there is a tendency that the frequency of contact with leukocytes is decreased so that the leukocytes are difficult to capture. In addition, the mechanical strength of the nonwoven fabric may be reduced.

The nonwoven fabric more suitable for carrying out the present embodiment may be defined by a filling rate. The filling rate of the nonwoven fabric is calculated according to the following expression (10) by measuring the area, thickness, and mass of the nonwoven fabric cut into an arbitrary dimension and the specific gravity of the material constituting the nonwoven fabric:

$$\text{Filling rate} = [\text{Mass (g) of the nonwoven fabric} / (\text{Area (cm}^2) \text{ of the nonwoven fabric} \times \text{Thickness (cm) of the nonwoven fabric})] / \text{Specific gravity (g/cm}^3) \text{ of the material constituting the nonwoven fabric} \dots (10).$$

The filling rate of the nonwoven fabric constituting the first nonwoven fabric layer according to the present embodiment is preferably 0.04 or larger and 0.40 or smaller and may be more preferably 0.08 or larger and 0.30 or smaller. If the filling rate is larger than 0.40, there is a tendency that the flow resistance of the nonwoven fabric is increased by the capture of aggregates, leukocytes, and the like, and clogging by blood cells is accordingly increased so that processing speed is decreased. On the other hand, if the filling rate is smaller than 0.04, aggregate capture performance might be reduced so that the nonwoven fabric of the second nonwoven fabric layer is clogged, resulting in a reduced filtration rate. In addition, the mechanical strength of the nonwoven fabric may be reduced.

The filling rate of the nonwoven fabric constituting the second nonwoven fabric layer is preferably from 0.04 to 0.40 and may be more preferably from 0.06 to 0.30, further preferably from 0.08 to 0.22. If the filling rate of the nonwoven fabric of the second nonwoven fabric layer is larger than 0.40, there is a tendency that the flow resistance of the nonwoven fabric is increased, and clogging by blood cells is accordingly increased so that processing speed is decreased down. On the other hand, if the filling rate is smaller than 0.04, there is a tendency that the frequency of contact with leukocytes and the like is decreased so that the leukocytes are difficult to capture. In addition, the mechanical strength of the nonwoven fabric may be reduced.

In the present embodiment, examples of the fiber material for the nonwoven fabric contained in the filter element may include, but are not limited to, polymer materials such as polyester, polyamide, polyacrylonitrile, polymethyl methacrylate, polyethylene, and polypropylene. Also, metal fibers may be partially used. Use of fibers made of such a synthetic polymer material in the filter element can prevent the degeneration of blood. More preferably, the respective nonwoven fabrics of the first nonwoven fabric layer and the second nonwoven fabric layer having a stable fiber diameter can be obtained by adopting fibers containing polyester. Among others, polyethylene terephthalate or polybutylene terephthalate is preferred because of having affinity for a blood product and stable wettability for blood.

In the present embodiment, the CWST (critical wetting surface tension) of the nonwoven fabric (or the nonwoven fabric coated with a coat layer) contained in the filter element is preferably 70 dyn/cm or larger, more preferably 85 dyn/cm or larger, further preferably 95 dyn/cm or larger. The nonwoven fabric having such a critical wetting surface tension secures stable wettability for blood and is thereby capable of efficiently performing leukocyte removal while allowing platelets in a blood product to pass therethrough.

The CWST refers to a value determined according to the following method: aqueous solutions of sodium hydroxide, calcium chloride, sodium nitrate, acetic acid, or ethanol differing in concentration are prepared such that the surface tension varies by from 2 to 4 dyn/cm. The surface tension (dyn/cm) of each aqueous solution thus obtained is from 94 to 115 for the aqueous sodium hydroxide solutions, from 90 to 94 for the aqueous calcium chloride solutions, from 75 to 87 for the aqueous sodium nitrate solutions, 72.4 for pure water, from 38 to 69 for the aqueous acetic acid solutions, and from 22 to 35 for the aqueous ethanol solutions ("Kagaku Binran (Handbook of Chemistry in English), Basics II", revised 2nd edition., edited by The Chemical Society of Japan, Maruzen Publishing Co., Ltd., 1975, p. 164). Ten drops each of the thus-obtained aqueous solutions having different surface tension by from 2 to 4 dyn/cm are placed on the nonwoven fabric in ascending order of surface tension, and left for 10 minutes. The case where 9 or more out of the 10 drops left for 10 minutes are absorbed by the nonwoven fabric is determined to be a wet state, while the case where less than 9 out of the 10 drops are absorbed is determined to be a non-wet state. In this way, the liquids are assayed on the nonwoven fabric in the ascending order of the surface tension. During this assay, the determination changes from wet state to non-wet state. In this respect, the CWST value of the nonwoven fabric is defined as an average value of the surface tension value of the last liquid for which the wet state is observed and the surface tension value of the first liquid for which the non-wet state is observed. For example, the CWST value of the nonwoven fabric that is wet by a liquid having a surface tension of 64 dyn/cm and is non-wet by a liquid having a surface tension of 66 dyn/cm is 65 dyn/cm.

A general blood processing filter is usually subjected to sterilization treatment by steam heating before use in order to prevent the contamination of a blood product with infectious substances. It has been revealed that the filtration performance of the filter may be reduced in this operation.

Such reduction in filtration performance is probably because:

1) The nonwoven fabric constituting the filter element contracts in the planar direction due to the steam heat treatment, whereby the filter element is unstably held by the container members so that a phenomenon in which blood leaks through the holding parts and runs into the outlet space from the inlet space without passing through the filter element (side leak phenomenon) occurs to thereby reduce filtration performance.

2) As with 1), the nonwoven fabric constituting the filter element contracts in the planar direction due to the steam heat treatment, whereby not only is the pore size of the nonwoven fabric decreased but the pore size becomes non-uniform to thereby increase clogging by blood cells and decrease processing speed. If extreme reduction in pore size occurs, blood flows only in a part of the filter element. This decreases an effective filtration area and reduces filtration performance.

3) The average fiber diameter of the nonwoven fabric constituting the filter element is decreased due to the steam heat treatment, whereby the surface area per unit mass of the filter element is reduced. This decreases an adsorption area for unnecessary components (leukocytes, etc.) and reduces filtration performance.

4) As with 3), the average fiber diameter of the nonwoven fabric constituting the filter element is decreased due to the steam heat treatment, whereby the mean flow pore size in the vertical direction of the filter element is increased. This reduces the number of contacts of the filter element with blood cells per unit time and thereby reduces filtration performance.

As mentioned above, change in the physical properties of the nonwoven fabric in association with the steam heat treatment may be reasons for largely deteriorating the performance balance of a blood processing filter.

In this context, the present inventor has conceived the idea that all the 4 points described above about change in the physical properties of the filter element can be detected beforehand from change in the airflow pressure drop of a filter. Specifically,

As described above in 1), the holding part of the filter becomes structurally unstable after steam heating and thereby facilitates the flow of air leaking therethrough. Therefore, the airflow pressure drop of the filter is reduced.

In the case of 2) described above, the pore size of the nonwoven fabric is decreased after steam heating, whereby the airflow resistance of the nonwoven fabric is increased. Therefore, the airflow pressure drop of the filter is increased.

In 3) and 4) described above, the average fiber diameter of the nonwoven fabric is decreased after steam heating, whereby airflow resistance is reduced with increase in the pore size of the nonwoven fabric. Therefore, the airflow pressure drop of the filter is reduced.

The change in airflow pressure drop shows a reverse trend in 1) and 2) described above. The respective degrees of contribution of 1) and 2) differ depending on the physical properties (e.g., average fiber diameter and bulk density) of the nonwoven fabric fiber, and they will not be canceled.

It has been further found that reduction in the filtration performance of the blood processing filter by sterilization by steam heating can be suppressed by adjusting change in the airflow pressure drop of the filter before and after exposure to steam of 115°C for 240 minutes to a predetermined level or lower (specifically, ±2% or less).

In this context, the airflow pressure drop serves as an index that indicates the resistance value of the filter against the flow of air. This index is suitable for evaluating filtration performance such as leukocyte removal performance.

The airflow pressure drop is determined by measuring the pressure drop (Pa) of air caused in the filter when dry air flows at a predetermined flow rate (3.0 ml/min) in the filter. The pressure drop can be measured using, for example, a DP gauge manufactured by Cosmo Solutions Technology Inc. (model: DP-320B). In the case of measuring the airflow pressure drop after exposure to steam of 115°C for 240 minutes, the filter thus exposed to steam is vacuum-dried at 40°C for 15 hours or longer, and then, the pressure drop value is determined.

The rate of change in airflow pressure drop before and after exposure to steam of 115°C for 240 minutes can be determined according to the following expression:

$$\text{Rate of change in airflow pressure drop (\%)} = (P_1 - P_0) / P_0 * 100$$

wherein $P_0$ represents an airflow pressure drop value before the exposure, and $P_1$ represents an airflow pressure drop value after the exposure.

As the airflow pressure drop of the filter is increased, there is a tendency that processing speed is decreased, and in an extreme case, filtration performance (leukocyte removal performance) is also reduced. On the other hand, as the airflow pressure drop is decreased, there is a tendency that leukocyte removal performance is reduced.

For suppressing reduction in the performance of the blood processing filter after sterilization by steam heating and achieving favorable performance balance, the rate of change in airflow pressure drop before and after exposure to steam of 115°C for 240 minutes is preferably ±2% or less. The rate of change in airflow pressure drop is more preferably ±1.5% or less, further preferably ±1.0% or less, particularly preferably ±0.5% or less.

Conditions for sterilization by steam heating to be actually performed differ variously depending on the kits incorporating the blood processing filter produced by each bag manufacturer. In the present embodiment, the blood processing filter having favorable performance balance even after sterilization by steam heating has been achieved by setting the standard conditions for the sterilization by steam heating to be exposure to steam of 115°C for 240 minutes, and controlling the rate of change in airflow pressure drop before and after exposure to steam of 115°C for 240 minutes to ±2% or less.

The reason why the performance balance of the filter after steam heating is improved by controlling the rate of change in airflow pressure drop before and after exposure to steam of 115°C for 240 minutes to ±2% or less is probably because reduction in the frequency of contact of the filter element with blood and the side leak of blood are suppressed by suppressing reduction in airflow pressure drop, and clogging during filtration of blood is suppressed by suppressing increase in airflow pressure drop. However, the mechanism is not limited thereto.

Part of the reason for the change in the airflow pressure drop of the filter by steam heat treatment is probably because the polymer material (e.g., polyester resin) of the nonwoven fabric constituting the filter element has insufficient crystallinity. In short, it is considered that a low crystalline polymer material is heat-treated at a high temperature equal to or higher than Tg so that the resin density in the nonwoven fabric is elevated due to the increased crystallization of the nonwoven fabric to thereby decrease the volume per unit mass of the nonwoven fabric, accordingly causing change in physical properties, such as reduction in average fiber diameter or contraction.

In the production of the nonwoven fabric contained in the filter element according to the present embodiment, for example, a nonwoven fabric having high crystallinity is produced by applying a sufficient quantity of heat thereto. Change in the physical properties of the resulting nonwoven fabric between before and after steam heating is suppressed. Thus, a filter with a little change in airflow pressure drop can be prepared.

The filter element thus prepared has thermally stable nature and therefore has heat stability that allows the rate of change in airflow pressure drop to be kept low under a wider range of steam heating conditions, as compared with conventional filter elements.

In the present embodiment, when the quantity of heat of the uncrystallized form of the nonwoven fabric contained in the filter element 5 is 5 J/g or smaller before exposure to steam of 100°C or higher, this nonwoven fabric is considered to have sufficient crystallinity. By controlling the quantity of heat of the uncrystallized form of the nonwoven fabric to 5 J/g or smaller, the change in the physical properties of the nonwoven fabric, such as contraction or reduction in average fiber diameter, in association with the increased crystallization of the nonwoven fabric in the filter during steam heating can be suppressed, and thus the rate of change in airflow pressure drop of the filter can be lowered.

When the value obtained by subtracting the quantity of heat of the uncrystallized form of the nonwoven fabric contained in the filter element from its quantity of heat of crystal melting is 50 J/g or larger before exposure to steam of 100°C or higher, this nonwoven fabric contained in the filter element has higher crystallinity. Therefore, the change in the physical properties (contraction, etc.) of the filter element between before and after steam heating is suppressed. Thus, the rate of change in airflow pressure drop can be further lowered.

In this context, the quantity of heat of the uncrystallized form and the quantity of heat of crystal melting are values evaluated as to the nonwoven fabric by differential scanning calorimetry (DSC). Such a measurement method will be described below.

From 3 to 4 mg of the nonwoven fabric is separated and loaded in an aluminum standard container. An initial heating curve (DSC curve) is measured at an initial temperature of 35°C at a heating rate of 10°C/min in an atmosphere of 50 mUmin nitrogen flow. An exothermic peak and a melting peak (endothermic peak) are detected from this initial heating curve (DSC curve). The values of quantity of heat (J) obtained from their respective peak areas are divided by the mass of the nonwoven fabric to calculate the quantity of heat of the uncrystallized form (J/g) and the quantity of heat of crystal melting (J/g). For example, TA-60WS system manufactured by Shimadzu Corp. can be used as a measurement apparatus.

In the present embodiment, the X-ray crystallinity of the nonwoven fabric contained in the filter element is preferably 60 or larger before exposure to steam of 100°C or higher. This further enhances the crystallinity of the nonwoven fabric contained in the filter element and suppresses the change in the physical properties (contraction, etc.) of the filter element between before and after steam heating. As a result, the effect of further reducing the rate of change in airflow pressure drop is obtained.

The X-ray crystallinity is more preferably 63 or larger, further preferably 66 or larger.

In the present embodiment, the X-ray crystallinity is measured by an X-ray diffraction method. The measurement can be performed by the following measurement steps 1) to 5) using an X-ray diffraction apparatus (e.g., MiniFlexll (Rigaku Corp., model 2005H301)):

1) One nonwoven fabric having a size of 3 cm × 3 cm is loaded on a sample table.
2) The sample is assayed under the following conditions:

Scanning range: from 5° to 50°
Sampling width (width for data fetch): 0.02°
Scan speed: 2.0°/min
Voltage: 30 kV
Current: 15 mA

3) After the assay, data with peaks from an amorphous part and a crystalline part being separated from each other is obtained.
4) An amorphous peak area (Aa) and a total peak area (At) are determined from the data of the step 3). The data obtained in the step 3) is analyzed with, for example, analytical software (MDI JADE 7) to carry out an "automatic peak separation" function. As a result, the amorphous peak area (Aa) and the total peak area (At) are automatically calculated.
5) The crystallinity is calculated according to the following expression from the amorphous peak area (Aa) and the total peak area (At):

$$\text{Crystallinity (\%)} = (At - Aa) / At \times 100$$

In the present embodiment, the area contraction rate of the nonwoven fabric exposed to steam of 115°C for 240 minutes is preferably 10% or smaller, more preferably 3% or smaller, particularly preferably 2% or smaller, most preferably 1% or smaller. If the contraction rate is larger than 10%, there is a tendency that, when severe steam heat treatment such as high-pressure steam sterilization is conducted, not only is the pore size of the nonwoven fabric decreased but the pore size becomes non-uniform to thereby increase clogging by blood cells and decrease processing speed. On the other hand, the area contraction rate of 10% or smaller is preferred because there is a tendency that the pore size is kept uniform even after steam heat treatment so that variation in processing speed can be prevented, and stable performance balance can be exerted.

In addition, the compressive force of the holding parts is improved after steam heat treatment and suppresses a phenomenon in which blood leaks through the holding parts and runs into the outlet space from the inlet space without passing through the filter element (side leak phenomenon). As a result, the effect of improving leukocyte removal performance is also obtained.

In this respect, polybutylene terephthalate has a faster crystallization speed than that of other polyester fibers, for example, polyethylene terephthalate fibers. Therefore, its crystallinity is easily elevated. The resulting nonwoven fabric is less likely to contract in the planar direction even by severe steam heat treatment such as high-pressure steam sterilization (the area contraction rate is easily decreased) and can thus exert stable removal performance for leukocytes and the like and processing speed.

In the present embodiment, the area contraction rate of the nonwoven fabric before and after exposure to steam of 115°C for 240 minutes is calculated according to the following expression (20) by accurately measuring the horizontal and vertical sizes of the nonwoven fabric cut into a square of approximately 20 cm × 20 cm, then exposing the nonwoven fabric to steam of 115°C for 240 minutes without fixing the nonwoven fabric with a pin or the like, and then measuring the horizontal and vertical sizes again:

Area contraction rate (%)

= (Vertical length (cm) of the nonwoven fabric before the steam

exposure × Horizontal length (cm) of the nonwoven fabric before the steam

exposure - Vertical length (cm) of the nonwoven fabric after the steam

exposure × Horizontal length (cm) of the nonwoven fabric after the steam

exposure) / (Vertical length (cm) of the nonwoven fabric before the steam

exposure × Horizontal length (cm) of the nonwoven fabric before the steam

exposure) × 100 ... (20).

The nonwoven fabric contained in the filter element of the present embodiment is not limited by its production method and can be produced by any of wet and dry methods at a spinning stage. In the present embodiment, the nonwoven fabric is produced by a melt blown method because a nonwoven fabric having the optimum formation index and average fiber diameter is stably obtained.

For preparing a filter with a little change in airflow pressure drop before and after exposure to steam of 115°C for 240 minutes, it is preferred to apply a sufficient quantity of heat to the nonwoven fabric constituting the filter element.

One example of the melt blown method will be described as the method for producing the nonwoven fabric used in the present embodiment. In the melt blown method, a molten polymer fluid obtained by melting in an extruder is filtered through an appropriate filter, then introduced to a molten polymer inlet of a melt blown die, and then discharged from an orifice nozzle. At the same time therewith, a heated gas introduced to a heated gas inlet is introduced to a heated gas ejection slit formed from the melt blown die and a lip, and ejected therefrom so that the discharged molten polymer is attenuated to form ultrathin fibers. The formed ultrathin fibers are laminated to thereby obtain a nonwoven fabric. The nonwoven fabric can be further heat-treated using a heat suction drum, a hot plate, hot water, a hot air heater, etc. to obtain a nonwoven fabric having the desired rebound strength.

In this respect, for sufficiently enhancing the rebound strength per unit basis weight of the nonwoven fabric, the heating temperature and time is adjusted according to the properties of the polymer. For producing the nonwoven fabric having high rebound strength per unit basis weight before steam heat treatment and furthermore, for preparing a filter with a little change in airflow pressure drop before and after exposure to steam of 115°C for 240 minutes, the temperature of the heat source is a temperature equal to or higher than [melting point of the polymer - 120]°C, preferably a temperature within a range from [melting point of the polymer - 20]°C to [melting point of the polymer - 60]°C. The heating time varies depending on the heating temperature and is at least 3 seconds or longer, preferably 10 seconds or longer, further preferably 20 seconds or longer, more preferably 30 seconds or longer.

On the other hand, if the temperature of the heat source is lower than [melting point of the polymer - 120]°C or if the heating time is shorter than 3 seconds, this is not preferred because the quantity of heat necessary for achieving the favorable rebound strength and rate of change in airflow pressure drop tends to be difficult to obtain.

As one example, the rebound strength and the rate of change in airflow pressure drop suitable for the present embodiment can be obtained by allowing the polybutylene terephthalate nonwoven fabric after spinning to stay in dry air of 140°C for 120 seconds.

The material for the container which houses the filter element may be any of rigid resins and flexible resins. Examples of the rigid resin material include phenol resin, acrylic resin, epoxy resin, formaldehyde resin, urea resin, silicon resin, ABS resin, nylon, polyurethane, polycarbonate, vinyl chloride, polyethylene, polypropylene, polyester, and styrene-butadiene copolymers.

The flexible resin material for the container is preferably similar in thermal and electrical properties to the filter element. Examples of suitable materials include: thermoplastic elastomers such as soft polyvinyl chloride, polyurethane, ethylene-vinyl acetate copolymers, polyolefins such as polyethylene and polypropylene, hydrogenation products of styrene-butadiene-styrene copolymers, and styrene-isoprene-styrene copolymers or hydrogenation products thereof; and mixtures of the thermoplastic elastomers with softening agents such as polyolefins and ethylene-ethyl acrylate. The material is preferably soft vinyl chloride, polyurethane, an ethylene-vinyl acetate copolymer, a polyolefin, or a thermoplastic elastomer composed mainly of any of them, more preferably soft vinyl chloride or a polyolefin.

The shape of the container is not particularly limited as long as the shape has an inlet for a liquid to be processed (leukocyte-containing liquid) and an outlet for a processed (leukocyte-free) liquid. The shape is preferably adapted to the shape of the filter element.

When the filter element is, for example, plate-like, the container can have a flat shape consisting of a polygon such as a tetragon or a hexagon, a circle, an ellipse, or the like according to the plate-like shape. More specific examples thereof include configuration in which, as shown in Figure 1 or 2, the container 1 is constituted by an inlet-side container member having the first port 3 as a liquid inlet/outlet and an outlet-side container member having the second port 4 as a liquid inlet/outlet, and both the container members sandwich the filter element 5 either directly or via a support such that the inside of the filter is divided into two rooms to form the flat blood processing filter 10.

As another example, when the filter element is cylindrical, it is preferred that the container should also be cylindrical. More specifically, the container is constituted by a tubular barrel which houses the filter element, an inlet-side header having a liquid inlet, and an outlet-side header having a liquid outlet, and preferably has a shape in which the inside of the container is divided into two spaces by potting such that a liquid introduced from the inlet flows from the outer periphery to the inner periphery (or from the inner periphery to the outer periphery) of the cylindrical filter, to form the cylindrical blood processing filter.

Next, a leukocyte removal method using the blood processing filter of the present embodiment will be described.

The leukocyte removal method of the present embodiment comprises allowing a leukocyte-containing liquid to pass through a blood processing filter having a filter element containing a nonwoven fabric housed in a container, to remove leukocytes from the leukocyte-containing liquid.

In this context, the leukocyte-containing liquid collectively includes body fluids and synthetic blood containing leukocytes, and specific examples include whole blood and a liquid consisting of a single or plural types of blood components obtained by preparation from whole blood, such as whole blood, a concentrated red cell solution, a washed red cell suspension, a thawed red cell concentrate, synthetic blood, platelet-poor plasma (PPP), platelet-rich plasma (PRP), plasma, frozen plasma, a platelet concentrate, and buffy coat (BC); a solution in which an anticoagulant, a preservative solution, or the like is added thereto; or a whole blood product, a red cell product, a platelet product, a plasma product, or the like.

Furthermore, a liquid obtained by processing the liquid mentioned above by the method of the present embodiment is referred to as a leukocyte-free liquid.

Hereinafter, one mode of a method for preparing each blood product by removing leukocytes by the leukocyte removal method will be described.

(Preparation of leukocyte-free whole blood product)

The leukocyte-free whole blood product can be obtained by providing a whole blood product by the addition of, for example, a preservative solution or an anticoagulant, such as citrate phosphate dextrose (CPD), citrate phosphate dextrose adenine-1 (CPDA-1), citrate phosphate-2-dextrose (CP2D), acid citrate dextrose formula-A (ACD-A), acid citrate dextrose formula-B (ACD-B), or heparin, to collected whole blood, and then removing leukocytes from the whole blood product using the blood processing filter of the present embodiment.

In the preparation of the leukocyte-free whole blood product, in the case of leukocyte removal before preservation, the whole blood preserved at room temperature or under refrigeration can be subjected to leukocyte removal using the blood processing filter at room temperature or under refrigeration preferably within 72 hours, more preferably within 24 hours, particularly preferably within 12 hours, most preferably within 8 hours after blood collection to obtain the leukocyte-free whole blood product. In the case of leukocyte removal after preservation, leukocytes can be removed from the whole blood preserved at room temperature, under refrigeration, or under freezing, preferably within 24 hours before use, using the blood processing filter to obtain the leukocyte-free whole blood product.

(Preparation of leukocyte-free red cell product)

A preservative solution or an anticoagulant, such as CPD, CPDA-1, CP2D, ACD-A, ACD-B, or heparin, is added to collected whole blood. A separation method for each blood component includes the case of performing centrifugation after removal of leukocytes from the whole blood, and the case of removing leukocytes from red cells or red cells and BC after centrifugation of the whole blood.

In the case of performing centrifugation after removal of leukocytes from the whole blood, the leukocyte-free red cell product can be obtained by centrifuging the leukocyte-free whole blood.

In the case of centrifuging the whole blood before leukocyte removal, the centrifugation conditions are divided into two types: soft spin conditions where the whole blood is separated into red cells and PRP, and hard spin conditions where the whole blood is separated into red cells, BC, and PPP. After addition of a preservative solution such as SAGM, AS-1, AS-3, AS-5, or MAP, if necessary, to red cells separated from the whole blood or red cells containing BC, leukocytes can be removed from the red cells using the blood processing filter to obtain the leukocyte-free red cell product.

In the preparation of the leukocyte-free red cell product, the whole blood preserved at room temperature or under refrigeration can be centrifuged preferably within 72 hours, more preferably within 48 hours, particularly preferably within 24 hours, most preferably within 12 hours after blood collection. In the case of leukocyte removal before preservation, leukocytes can be removed from the red cell product preserved at room temperature or under refrigeration, preferably within 120 hours, more preferably within 72 hours, particularly preferably within 24 hours, most preferably within 12 hours after blood collection, using the blood processing filter at room temperature or under refrigeration to obtain the leukocyte-free red cell product. In the case of leukocyte removal after preservation, leukocytes can be removed from the red cell product preserved at room temperature, under refrigeration, or under freezing, preferably within 24 hours before use, using the blood processing filter to obtain the leukocyte-free red cell product.

(Preparation of leukocyte-free platelet product)

A preservative solution or an anticoagulant, such as CPD, CPDA-1, CP2D, ACD-A, ACD-B, or heparin, is added to collected whole blood.

A separation method for each blood component includes the case of performing centrifugation after removal of leukocytes from the whole blood, and the case of removing leukocytes from PRP or platelet after centrifugation of the whole blood.

In the case of performing centrifugation after removal of leukocytes from the whole blood, the leukocyte-free platelet product can be obtained by centrifuging the leukocyte-free whole blood.

In the case of centrifuging the whole blood before leukocyte removal, the centrifugation conditions are divided into two types: soft spin conditions where the whole blood is separated into red cells and PRP, and hard spin conditions where the whole blood is separated into red cells, BC, and PPP. Under the soft spin conditions, leukocytes are removed from PRP separated from the whole blood with the blood processing filter, and then, the leukocyte-free platelet product is obtained by centrifugation, or platelet and PPP are obtained by centrifuging PRP, and then, leukocytes can be removed with the blood processing filter to obtain the leukocyte-free platelet product. Under the hard spin conditions, a pool of one unit or several to dozen units of BC separated from the whole blood is supplemented, if necessary, with a preservative solution, plasma, or the like, and centrifuged to obtain platelet, and leukocytes can be removed from the obtained platelet with the blood processing filter to obtain the leukocyte-free platelet product.

In the preparation of the leukocyte-free platelet product, the whole blood preserved at room temperature is centrifuged preferably within 24 hours, more preferably within 12 hours, particularly preferably within 8 hours after blood collection. In the case of leukocyte removal before preservation, leukocytes can be removed from the platelet product preserved at room temperature, preferably within 120 hours, more preferably within 72 hours, particularly preferably within 24 hours, most preferably within 12 hours after blood collection, using the blood processing filter at room temperature to obtain the leukocyte-free platelet product. In the case of leukocyte removal after preservation, leukocytes can be removed from the platelet product preserved at room temperature, under refrigeration, or under freezing, preferably within 24 hours before use, using the blood processing filter to obtain the leukocyte-free platelet product.

(Preparation of leukocyte-free plasma product)

A preservative solution or an anticoagulant, such as CPD, CPDA-1, CP2D, ACD-A, ACD-B, or heparin, is added to collected whole blood.

A separation method for each blood component includes the case of performing centrifugation after removal of leukocytes from the whole blood, and the case of removing leukocytes from PPP or PRP after centrifugation of the whole blood.

In the case of performing centrifugation after removal of leukocytes from the whole blood, the leukocyte-free plasma product can be obtained by centrifuging the leukocyte-free whole blood.

In the case of centrifuging the whole blood before leukocyte removal, the centrifugation conditions are divided into two types: soft spin conditions where the whole blood is separated into red cells and PRP, and hard spin conditions where the whole blood is separated into red cells, BC, and PPP. Under the soft spin conditions, leukocytes are removed from PRP with the blood processing filter, and then, the leukocyte-free plasma product is obtained by centrifugation, or PRP is centrifuged into PPP and platelet, and then, leukocytes can be removed with the blood processing filter to obtain the leukocyte-free plasma product. Under the hard spin conditions, leukocytes can be removed from PPP with the blood processing filter to obtain the leukocyte-free plasma product.

In the preparation of the leukocyte-free plasma product, the whole blood preserved at room temperature or under refrigeration can be centrifuged preferably within 72 hours, more preferably within 48 hours, particularly preferably within 24 hours, most preferably within 12 hours after blood collection. Leukocytes can be removed from the plasma product preserved at room temperature or under refrigeration, preferably within 120 hours, more preferably within 72 hours, particularly preferably within 24 hours, most preferably within 12 hours after blood collection, using the blood processing filter at room temperature or under refrigeration to obtain the leukocyte-free plasma product. In the case of leukocyte removal after preservation, leukocytes can be removed from the plasma product preserved at room temperature, under refrigeration, or under freezing, preferably within 24 hours before use, using the blood processing filter to obtain the leukocyte-free plasma product.

Any mode such as a mode of collecting blood with a blood collection needle connected with a container for whole blood, and connecting the container containing whole blood or blood components after centrifugation with the blood processing filter, followed by leukocyte removal, a mode of collecting blood using a circuit in which at least a blood collection needle, a blood container, and the blood processing filter are sterilely connected, and performing leukocyte removal before centrifugation or after centrifugation, or a mode of connecting the blood processing filter with a container containing blood components obtained in an automatic blood collection apparatus or using the blood processing filter connected in advance with the container to perform leukocyte removal may be used as a mode from blood collection to the preparation of a leukocyte-free blood product, though the present embodiment is not limited by these modes. Alternatively, the leukocyte-free red cell product, the leukocyte-free platelet product, or the leukocyte-free plasma product may be obtained by centrifuging whole blood into each component in an automatic blood component collection apparatus, if necessary adding a preservative solution, and immediately thereafter allowing any of red cells, BC-containing red cells, BC, platelet, PRP, and PPP to pass through the blood processing filter to remove leukocytes.

The method of the present embodiment has higher leukocyte removal performance for all types of blood described above and is effective for shortening a processing time without causing clogging. The method of the present embodiment is particularly suitable for processing a red cell-containing liquid, which is prone to extend a blood processing time.

In the preparation of these blood products, the leukocyte removal may be performed by allowing the leukocyte-containing blood to flow from a container located at a position higher than the blood processing filter into the blood processing filter via a tube, or by allowing the leukocyte-containing blood to flow by increasing pressure from the inlet side of the blood processing filter and/or reducing pressure from the outlet side of the blood processing filter using means such as a pump.

Hereinafter, a leukocyte removal method using the blood processing filter for extracorporeal circulation therapy will be described.

The inside of the blood processing filter is primed with physiological saline or the like, which is then replaced with a solution containing an anticoagulant such as heparin, nafamostat mesilate, ACD-A, or ACD-B. While the anticoagulant is added to blood diverted outside the body, the blood is injected into the inlet of the blood processing filter from a circuit connected with a human at a flow rate of from 10 to 200 mL/min, and leukocytes can be removed with the blood processing filter.

In the initial period of leukocyte removal (throughput: from 0 to 0.5 L), the flow rate is preferably from 10 to 50 mL/min, more preferably from 20 to 40 mL/min. After the initial period of leukocyte removal (throughput: from 0.2 to 12 L), the blood is preferably processed at a flow rate of from 30 to 120 mL/min, more preferably from 40 to 100 mL/min, particularly preferably from 40 to 60 mL/min. It is preferred to substitute the inside of the blood processing filter with physiological saline or the like after the leukocyte removal to return the blood, because the blood within the blood processing filter is not wasted.

Examples

Hereinafter, the present invention will be described with reference to Examples.

[Example 1]

(Preparation of nonwoven fabric)

The nonwoven fabric used was a nonwoven fabric having a basis weight of 22 g/m$^2$, a thickness of 0.13 mm, a filling rate of 0.12, an average fiber diameter of 1.0 $\mu$m, a WC value of 2.1 (gf·cm/cm$^2$), and an area contraction rate of 1.3%, which was prepared by a method of spinning polyethylene terephthalate (hereinafter, abbreviated to PET) by the melt blown method to form a fiber assembly, followed by the dry heat treatment of the obtained fiber assembly at 140°C for 120 seconds.

The nonwoven fabric was further coated with a hydrophilic polymer by a method described below.

A copolymer of 2-hydroxyethyl methacrylate (hereinafter, abbreviated to HEMA) and diethylaminoethyl methacrylate (hereinafter, abbreviated to DEAMA) was synthesized by conventional solution radical polymerization. The polymerization reaction was performed at a monomer concentration of 1 mol/L in ethanol at 60°C for 8 hours in the presence of 1/200 mol of azoisobutyronitrile (AIBN) as an initiator. The nonwoven fabric was dipped in the ethanol solution of the obtained hydrophilic polymer. The absorbed redundant polymer solution was squeezed out of the nonwoven fabric removed from the polymer solution, and the polymer solution was dried off while dry air was sent, to form a coat layer covering the surface of the nonwoven fabric. The molar ratio of the nonionic group to the basic nitrogen-containing functional group in the surface portion (surface portion of the coat layer) of the nonwoven fabric coated with the polymer coat layer was 32.3. The mass of the coat layer per gram of the nonwoven fabric coated with the polymer coat layer was 9.0 mg/g (nonwoven fabric + coat layer). The CWST value was 100 dyn/cm.

(Preparation of filter for blood processing)

A rigid container was packed with 64 stacked sheets of the thus-obtained nonwoven fabric coated with the coat layer, and ultrasonic welding was conducted to prepare a filter having an effective filtration area of 45 cm$^2$.

The rebound strength per unit basis weight of the obtained filter element was 0.40 (N·m$^2$/g). This filter was steam-sterilized at 115°C for 240 minutes and then vacuum-dried at 40°C for 15 hours or longer to prepare a steam-sterilized filter. As a result of measuring the airflow pressure drop before and after the steam sterilization (exposure to steam of 115°C for 240 minutes), the rate of change in airflow pressure drop was 1.8%.

(Leukocyte removal performance evaluation)

Next, a testing method to evaluate leukocyte removal performance will be described. The blood used in evaluation was whole blood prepared by adding 70 mL of an anticoagulant CPD solution to 500 mL of blood immediately after blood collection, mixing them, and leaving the mixture standing for 2 hours. Hereinafter, this blood prepared for blood evaluation is referred to as pre-filtration blood.

A blood bag packed with the pre-filtration blood was connected with the inlet of the steam-sterilized filter through a 40 cm polyvinyl chloride tube having an inside diameter of 3 mm and an outside diameter of 4.2 mm. Further, a blood bag for recovery was similarly connected with the outlet of the filter via a 60 cm polyvinyl chloride tube having an inside diameter of 3 mm and an outside diameter of 4.2 mm. Then, the pre-filtration blood was allowed to flow from the bottom of the blood bag packed with the pre-filtration blood into the filter by means of the 100cm difference in height. The filtration time was measured until the amount of the blood flowing into the recovery bag became 0.5 g/min.

3 mL of blood (hereinafter, referred to as post-filtration blood) was further recovered from the recovery bag. The leukocyte removal performance was evaluated by determining a leukocyte residual rate. The leukocyte residual rate was calculated according to the following expression (30) by measuring the number of leukocytes in the pre-filtration blood and the post-filtration blood using flow cytometry (apparatus: FACSCanto manufactured by Becton, Dickinson and Company):

$$\text{Leukocyte residual rate} = [\text{Leukocyte concentration (number/}\mu\text{L) (post-filtration blood)}] / [\text{Leukocyte concentration (number/}\mu\text{L) (pre-filtration blood)}] \ ... \ (30).$$

The measurement of the number of leukocytes was performed by sampling 100 $\mu$L of each blood and using Leucocount kit (Becton, Dickinson and Company, Japan) containing beads.

In the case of conducting evaluation under the filter shape conditions described above (64 sheets of the nonwoven fabric, effective filtration area: 45 cm2), a leukocyte removal filter element that can achieve a filtration time of 30 minutes or shorter and a leukocyte residual rate of $10.0 \times 10^{-3}$ or less is regarded as being practically desirable. Since, at a leukocyte residual rate of $10^{-4}$ or less, the number of residual leukocytes is close to the measurement limit, the filter shape conditions were set as described above so as to attain the leukocyte residual rate of $10^{-4}$ or less. A filter element having performance that satisfies the filtration time of 30 minutes or shorter and the leukocyte residual rate of $10.0 \times 10^{-3}$ or less under the conditions described above can achieve a filter with a leukocyte residual rate of from $10^{-4}$ to $10^{-6}$ which is necessary for preventing severe adverse reactions, when it is designed suitably for actual use .

As a result, the leukocyte residual rate was $0.7 \times 10^{-3}$, and the filtration time was 19 minutes, demonstrating low blood process pressure and high leukocyte removal performance.

[Example 2]

The nonwoven fabric used was made of PET fibers and had a basis weight of 22 g/m$^2$, a thickness of 0.13 mm, a filling rate of 0.12, an average fiber diameter of 1.0 $\mu$m, a WC value of 1.7 (gf·cm/cm$^2$), and an area contraction rate of 1.1%. The nonwoven fabric was prepared by the method involving the dry heat treatment of a fiber assembly after spinning in the same way as in Example 1. Polymer coating treatment was performed in the same way as in Example 1 to form a coat layer covering the PET fibers. The CWST value after the polymer coating treatment was 100 dyn/cm.

The nonwoven fabric thus polymer-coated was used as a blood processing filter element. The rebound strength per unit basis weight of this filter element was 0.40 (N·m$^2$/g).

A filter was prepared in the same way as in Example 1. As a result, the rate of change in airflow pressure drop before and after steam sterilization was 1.6%. As a result of conducting the blood test, the leukocyte residual rate was $0.4 \times 10^{-3}$, and the filtration time was 17 minutes, demonstrating low blood process pressure and high leukocyte removal performance.

[Example 3]

The nonwoven fabric used was made of PET fibers and had a basis weight of 22 g/m$^2$, a thickness of 0.13 mm, a filling rate of 0.12, an average fiber diameter of 1.0 $\mu$m, a WC value of 2.1 (gf·cm/cm$^2$), and an area contraction rate of 1.5%. The nonwoven fabric was prepared by the method involving the dry heat treatment of a fiber assembly after spinning in the same way as in Example 1. Polymer coating treatment was performed in the same way as in Example 1 to form a coat layer covering the PET fibers. The CWST value after the polymer coating treatment was 100 dyn/cm. The nonwoven fabric thus polymer-coated was used as a blood processing filter element. The rebound strength per unit basis weight of this filter element was 0.31 (N·m$^2$/g).

A filter was prepared in the same way as in Example 1. As a result, the rate of change in airflow pressure drop before and after steam sterilization was 2.0%. As a result of conducting the blood test, the leukocyte residual rate was $5.2 \times 10^{-3}$, and the filtration time was 19 minutes, demonstrating low blood process pressure and high leukocyte removal performance.

[Example 4]

The nonwoven fabric used was made of PET fibers and had a basis weight of 22 g/m$^2$, a thickness of 0.13 mm, a filling rate of 0.12, an average fiber diameter of 1.0 $\mu$m, a WC value of 1.7 (gf·cm/cm$^2$), and an area contraction rate of 1.4%. The nonwoven fabric was prepared by the method involving the dry heat treatment of a fiber assembly after spinning in the same way as in Example 1. Polymer coating treatment was performed in the same way as in Example 1 to form a coat layer covering the PET fibers. The CWST value after the polymer coating treatment was 100 dyn/cm. The nonwoven fabric thus polymer-coated was used as a leukocyte removal filter element. The rebound strength per unit basis weight of this filter element was 0.31 (N·m$^2$/g).

A filter was prepared in the same way as in Example 1. As a result, the rate of change in airflow pressure drop before and after steam sterilization was 1.9%. As a result of conducting the blood test, the leukocyte residual rate was $4.1 \times 10^{-3}$, and the filtration time was 18 minutes, demonstrating low blood process pressure and high leukocyte removal performance.

[Example 5]

The nonwoven fabric used was made of PET fibers and had a basis weight of 22 g/m$^2$, a thickness of 0.13 mm, a filling rate of 0.12, an average fiber diameter of 1.0 $\mu$m, a WC value of 2.1 (gf·cm/cm$^2$), and an area contraction rate of 1.3%. The nonwoven fabric was prepared by the method involving the dry heat treatment of a fiber assembly after spinning in the same way as in Example 1. The nonwoven fabric was not subjected to polymer coating treatment. The rebound strength per unit basis weight of this filter element was 0.40 (N·m$^2$/g).

A filter was prepared in the same way as in Example 1. As a result, the rate of change in airflow pressure drop before and after steam sterilization was 1.8%. As a result of conducting the blood test, the leukocyte residual rate was $3.3 \times 10^{-3}$, and the filtration time was 22 minutes, demonstrating low blood process pressure and high leukocyte removal performance.

[Example 6]

The nonwoven fabric used was made of PET fibers and had a basis weight of 22 g/m$^2$, a thickness of 0.13 mm, a filling rate of 0.12, an average fiber diameter of 1.0 $\mu$m, a WC value of 1.7 (gf·cm/cm$^2$), and an area contraction rate of 1.1%. The nonwoven fabric was prepared by the method involving the dry heat treatment of a fiber assembly after spinning in the same way as in Example 1. The nonwoven fabric was not subjected to polymer coating treatment. The rebound strength per unit basis weight of this filter element was 0.40 (N·m$^2$/g).

A filter was prepared in the same way as in Example 1. As a result, the rate of change in airflow pressure drop before and after steam sterilization was 1.6%. As a result of conducting the blood test, the leukocyte residual rate was $2.8 \times 10^{-3}$, and the filtration time was 21 minutes, demonstrating low blood process pressure and high leukocyte removal performance.

[Example 7]

The nonwoven fabric used was made of PET fibers and had a basis weight of 22 g/m$^2$, a thickness of 0.13 mm, a filling rate of 0.12, an average fiber diameter of 1.0 $\mu$m, a WC value of 2.1 (gf·cm/cm$^2$), and an area contraction rate of 1.5%. The nonwoven fabric was prepared by the method involving the dry heat treatment of a fiber assembly after spinning in the same way as in Example 1. The nonwoven fabric was not subjected to polymer coating treatment. The rebound strength per unit basis weight of this filter element was 0.31 (N·m$^2$/g).

A filter was prepared in the same way as in Example 1. As a result, the rate of change in airflow pressure drop before and after steam sterilization was 2.0%. As a result of conducting the blood test, the leukocyte residual rate was 8.3 × 10$^{-3}$, and the filtration time was 23 minutes, demonstrating low blood process pressure and high leukocyte removal performance.

[Example 8]

The nonwoven fabric used was made of PET fibers and had a basis weight of 22 g/m$^2$, a thickness of 0.13 mm, a filling rate of 0.12, an average fiber diameter of 1.0 $\mu$m, a WC value of 1.7 (gf·cm/cm$^2$), and an area contraction rate of 1.4%. The nonwoven fabric was prepared by the method involving the dry heat treatment of a fiber assembly after spinning in the same way as in Example 1. The nonwoven fabric was not subjected to polymer coating treatment. The rebound strength per unit basis weight of this filter element was 0.31 (N·m$^2$/g).

A filter was prepared in the same way as in Example 1. As a result, the rate of change in airflow pressure drop before and after steam sterilization was 1.9%. As a result of conducting the blood test, the leukocyte residual rate was 7.3 × 10$^{-3}$, and the filtration time was 20 minutes, demonstrating low blood process pressure and high leukocyte removal performance.

[Example 9]

The nonwoven fabric used was made of polybutylene terephthalate (hereinafter, abbreviated to PBT) fibers and had a basis weight of 22 g/m$^2$, a thickness of 0.13 mm, a filling rate of 0.12, an average fiber diameter of 1.0 $\mu$m, a WC value of 2.1 (gf·cm/cm$^2$), and an area contraction rate of 1.2%. The nonwoven fabric was prepared by the method involving the dry heat treatment of a fiber assembly after spinning in the same way as in Example 1. Polymer coating treatment was performed in the same way as in Example 1 to form a coat layer covering the PBT fibers. The CWST value after the polymer coating treatment was 98 dyn/cm. The nonwoven fabric thus polymer-coated was used as a leukocyte removal filter element. The rebound strength per unit basis weight of this filter element was 0.40 (N·m$^2$/g).

A filter was prepared in the same way as in Example 1. As a result, the rate of change in airflow pressure drop before and after steam sterilization was 1.7%. As a result of conducting the blood test, the leukocyte residual rate was 0.5 × 10$^{-3}$, and the filtration time was 19 minutes, demonstrating low blood process pressure and high leukocyte removal performance.

[Example 10]

The nonwoven fabric used was made of PBT fibers and had a basis weight of 22 g/m$^2$, a thickness of 0.13 mm, a filling rate of 0.12, an average fiber diameter of 1.0 $\mu$m, a WC value of 1.7 (gf·cm/cm$^2$), and an area contraction rate of 1.0%. The nonwoven fabric was prepared by the method involving the dry heat treatment of a fiber assembly after spinning in the same way as in Example 1. Polymer coating treatment was performed in the same way as in Example 1 to form a coat layer covering the PBT fibers. The CWST value after the polymer coating treatment was 98 dyn/cm. The nonwoven fabric thus polymer-coated was used as a leukocyte removal filter element. The rebound strength per unit basis weight of this filter element was 0.40 (N·m$^2$/g).

A filter was prepared in the same way as in Example 1. As a result, the rate of change in airflow pressure drop before and after steam sterilization was 1.5%. As a result of conducting the blood test, the leukocyte residual rate was 0.1 × 10$^{-3}$, and the filtration time was 18 minutes, demonstrating low blood process pressure and high leukocyte removal performance.

[Example 11]

The nonwoven fabric used was made of PBT fibers and had a basis weight of 22 g/m$^2$, a thickness of 0.13 mm, a filling rate of 0.12, an average fiber diameter of 1.0 $\mu$m, a WC value of 2.1 (gf·cm/cm$^2$), and an area contraction rate of 1.4%. The nonwoven fabric was prepared by the method involving the dry heat treatment of a fiber assembly after spinning in the same way as in Example 1. Polymer coating treatment was performed in the same way as in Example 1 to form a coat layer covering the PBT fibers. The CWST value after the polymer coating treatment was 98 dyn/cm. The nonwoven fabric thus polymer-coated was used as a leukocyte removal filter element. The rebound strength per unit basis weight of this filter element was 0.31 (N·m²/g).

A filter was prepared in the same way as in Example 1. As a result, the rate of change in airflow pressure drop before and after steam sterilization was 1.9%. As a result of conducting the blood test, the leukocyte residual rate was $4.5 \times 10^{-3}$, and the filtration time was 19 minutes, demonstrating low blood process pressure and high leukocyte removal performance.

[Example 12]

The nonwoven fabric used was made of PBT fibers and had a basis weight of 22 g/m², a thickness of 0.13 mm, a filling rate of 0.12, an average fiber diameter of 1.0 μm, a WC value of 1.7 (gf·cm/cm²), and an area contraction rate of 1.3%. The nonwoven fabric was prepared by the method involving the dry heat treatment of a fiber assembly after spinning in the same way as in Example 1. Polymer coating treatment was performed in the same way as in Example 1 to form a coat layer covering the PBT fibers. The CWST value after the polymer coating treatment was 98 dyn/cm. The nonwoven fabric thus polymer-coated was used as a leukocyte removal filter element. The rebound strength per unit basis weight of this filter element was 0.31 (N·m²/g).

A filter was prepared in the same way as in Example 1. As a result, the rate of change in airflow pressure drop before and after steam sterilization was 1.8%. As a result of conducting the blood test, the leukocyte residual rate was $3.8 \times 10^{-3}$, and the filtration time was 20 minutes, demonstrating low blood process pressure and high leukocyte removal performance.

[Example 13]

The nonwoven fabric used was made of PBT fibers and had a basis weight of 22 g/m², a thickness of 0.13 mm, a filling rate of 0.12, an average fiber diameter of 1.0 μm, a WC value of 2.1 (gf·cm/cm²), and an area contraction rate of 1.2%. The nonwoven fabric was prepared by the method involving the dry heat treatment of a fiber assembly after spinning in the same way as in Example 1. The nonwoven fabric was not subjected to polymer coating treatment. The rebound strength per unit basis weight of this filter element was 0.40 (N·m²/g).

A filter was prepared in the same way as in Example 1. As a result, the rate of change in airflow pressure drop before and after steam sterilization was 1.7%. As a result of conducting the blood test, the leukocyte residual rate was $2.8 \times 10^{-3}$, and the filtration time was 29 minutes, demonstrating low blood process pressure and high leukocyte removal performance.

[Example 14]

The nonwoven fabric used was made of PBT fibers and had a basis weight of 22 g/m², a thickness of 0.13 mm, a filling rate of 0.12, an average fiber diameter of 1.0 μm, a WC value of 1.7 (gf·cm/cm²), and an area contraction rate of 1.0%. The nonwoven fabric was prepared by the method involving the dry heat treatment of a fiber assembly after spinning in the same way as in Example 1. The nonwoven fabric was not subjected to polymer coating treatment. The rebound strength per unit basis weight of this filter element was 0.40 (N·m²/g).

A filter was prepared in the same way as in Example 1. As a result, the rate of change in airflow pressure drop before and after steam sterilization was 1.5%. As a result of conducting the blood test, the leukocyte residual rate was $2.4 \times 10^{-3}$, and the filtration time was 28 minutes, demonstrating low blood process pressure and high leukocyte removal performance.

[Example 15]

The nonwoven fabric used was made of PBT fibers and had a basis weight of 22 g/m², a thickness of 0.13 mm, a filling rate of 0.12, an average fiber diameter of 1.0 μm, a WC value of 2.1 (gf·cm/cm²), and an area contraction rate of 1.4%. The nonwoven fabric was prepared by the method involving the dry heat treatment of a fiber assembly after spinning in the same way as in Example 1. The nonwoven fabric was not subjected to polymer coating treatment. The rebound strength per unit basis weight of this filter element was 0.31 (N·m²/g).

A filter was prepared in the same way as in Example 1. As a result, the rate of change in airflow pressure drop before and after steam sterilization was 1.9%. As a result of conducting the blood test, the leukocyte residual rate was $7.9 \times 10^{-3}$, and the filtration time was 29 minutes, demonstrating low blood process pressure and high leukocyte removal performance.

[Example 16]

The nonwoven fabric used was made of PBT fibers and had a basis weight of 22 g/m$^2$, a thickness of 0.13 mm, a filling rate of 0.12, an average fiber diameter of 1.0 $\mu$m, a WC value of 1.7 (gf·cm/cm$^2$), and an area contraction rate of 1.3%. The nonwoven fabric was prepared by the method involving the dry heat treatment of a fiber assembly after spinning in the same way as in Example 1. The nonwoven fabric was not subjected to polymer coating treatment. The rebound strength per unit basis weight of this filter element was 0.31 (N·m$^2$/g).
A filter was prepared in the same way as in Example 1. As a result, the rate of change in airflow pressure drop before and after steam sterilization was 1.8%. As a result of conducting the blood test, the leukocyte residual rate was $6.8 \times 10^{-3}$, and the filtration time was 29 minutes, demonstrating low blood process pressure and high leukocyte removal performance.

[Comparative Example 1]

The nonwoven fabric used was made of PET fibers and had a basis weight of 22 g/m$^2$, a thickness of 0.13 mm, a filling rate of 0.12, an average fiber diameter of 1.0 $\mu$m, a WC value of 2.1 (gf·cm/cm$^2$), and an area contraction rate of 2.7%. The nonwoven fabric was prepared by the method involving the dry heat treatment of a fiber assembly after spinning in the same way as in Example 1. Polymer coating treatment was performed in the same way as in Example 1 to form a coat layer covering the PET fibers. The CWST value after the polymer coating treatment was 100 dyn/cm. The nonwoven fabric thus polymer-coated was used as a leukocyte removal filter element. The rebound strength per unit basis weight of this filter element was 0.28 (N·m$^2$/g).
A filter was prepared in the same way as in Example 1. As a result, the rate of change in airflow pressure drop before and after steam sterilization was 1.7%. As a result of conducting the blood test, the leukocyte residual rate was $16.5 \times 10^{-3}$, and the filtration time was 21 minutes, demonstrating that this filter element was practically unsuitable due to low leukocyte removal performance, though its filtration time was short.

[Comparative Example 2]

The nonwoven fabric used was made of PET fibers and had a basis weight of 22 g/m$^2$, a thickness of 0.13 mm, a filling rate of 0.12, an average fiber diameter of 1.0 $\mu$m, a WC value of 1.7 (gf·cm/cm$^2$), and an area contraction rate of 1.6%. The nonwoven fabric was prepared by the method involving the dry heat treatment of a fiber assembly after spinning in the same way as in Example 1. Polymer coating treatment was performed in the same way as in Example 1 to form a coat layer covering the PET fibers. The CWST value after the polymer coating treatment was 100 dyn/cm. The nonwoven fabric thus polymer-coated was used as a leukocyte removal filter element. The rebound strength per unit basis weight of this filter element was 0.28 (N·m$^2$/g).
A filter was prepared in the same way as in Example 1. As a result, the rate of change in airflow pressure drop before and after steam sterilization was 1.6%. As a result of conducting the blood test, the leukocyte residual rate was $13.8 \times 10^{-3}$, and the filtration time was 20 minutes, demonstrating that this filter element was practically unsuitable due to low leukocyte removal performance, though its filtration time was short.

[Comparative Example 3]

The nonwoven fabric used was made of PET fibers and had a basis weight of 22 g/m$^2$, a thickness of 0.13 mm, a filling rate of 0.12, an average fiber diameter of 1.0 $\mu$m, a WC value of 2.1 (gf·cm/cm$^2$), and an area contraction rate of 2.7%. The nonwoven fabric was prepared by the method involving the dry heat treatment of a fiber assembly after spinning in the same way as in Example 1. The nonwoven fabric was not subjected to polymer coating treatment. The rebound strength per unit basis weight of this filter element was 0.28 (N·m$^2$/g).
A filter was prepared in the same way as in Example 1. As a result, the rate of change in airflow pressure drop before and after steam sterilization was 1.7%. As a result of conducting the blood test, the leukocyte residual rate was $19.5 \times 10^{-3}$, and the filtration time was 29 minutes, demonstrating that this filter element was practically unsuitable due to low leukocyte removal performance, though its filtration time was short.

[Comparative Example 4]

The nonwoven fabric used was made of PET fibers and had a basis weight of 22 g/m$^2$, a thickness of 0.13 mm, a filling rate of 0.12, an average fiber diameter of 1.0 $\mu$m, a WC value of 1.7 (gf·cm/cm$^2$), and an area contraction rate of 1.6%. The nonwoven fabric was prepared by the method involving the dry heat treatment of a fiber assembly after spinning in the same way as in Example 1. The nonwoven fabric was not subjected to polymer coating treatment. The rebound strength per unit basis weight of this filter element was 0.28 (N·m$^2$/g).

A filter was prepared in the same way as in Example 1. As a result, the rate of change in airflow pressure drop before and after steam sterilization was 1.6%. As a result of conducting the blood test, the leukocyte residual rate was 17.4 × 10$^{-3}$, and the filtration time was 30 minutes, demonstrating that this filter element was practically unsuitable due to low leukocyte removal performance, though its filtration time was acceptable.

[Comparative Example 5]

The nonwoven fabric used was made of PBT fibers and had a basis weight of 22 g/m$^2$, a thickness of 0.13 mm, a filling rate of 0.12, an average fiber diameter of 1.0 μm, a WC value of 2.1 (gf·cm/cm$^2$), and an area contraction rate of 2.6%. The nonwoven fabric was prepared by the method involving the dry heat treatment of a fiber assembly after spinning in the same way as in Example 1. Polymer coating treatment was performed in the same way as in Example 1 to form a coat layer covering the PBT fibers. The CWST value after the polymer coating treatment was 98 dyn/cm. The nonwoven fabric thus polymer-coated was used as a leukocyte removal filter element. The rebound strength per unit basis weight of this filter element was 0.28 (N·m$^2$/g).

A filter was prepared in the same way as in Example 1. As a result, the rate of change in airflow pressure drop before and after steam sterilization was 1.6%. As a result of conducting the blood test, the leukocyte residual rate was 13.5 × 10$^{-3}$, and the filtration time was 22 minutes, demonstrating that this filter element was practically unsuitable due to low leukocyte removal performance, though its filtration time was short.

[Comparative Example 6]

The nonwoven fabric used was made of PBT fibers and had a basis weight of 22 g/m$^2$, a thickness of 0.13 mm, a filling rate of 0.12, an average fiber diameter of 1.0 μm, a WC value of 1.7 (gf·cm/cm$^2$), and an area contraction rate of 1.5%. The nonwoven fabric was prepared by the method involving the dry heat treatment of a fiber assembly after spinning in the same way as in Example 1. Polymer coating treatment was performed in the same way as in Example 1 to form a coat layer covering the PBT fibers. The CWST value after the polymer coating treatment was 98 dyn/cm. The nonwoven fabric thus polymer-coated was used as a leukocyte removal filter element. The rebound strength per unit basis weight of this filter element was 0.28 (N·m$^2$/g).

A filter was prepared in the same way as in Example 1. As a result, the rate of change in airflow pressure drop before and after steam sterilization was 1.4%. As a result of conducting the blood test, the leukocyte residual rate was 12.1 × 10$^{-3}$, and the filtration time was 23 minutes, demonstrating that this filter element was practically unsuitable due to low leukocyte removal performance, though its filtration time was short.

[Comparative Example 7]

The nonwoven fabric used was made of PBT fibers and had a basis weight of 22 g/m$^2$, a thickness of 0.13 mm, a filling rate of 0.12, an average fiber diameter of 1.0 μm, a WC value of 2.1 (gf·cm/cm$^2$), and an area contraction rate of 2.6%. The nonwoven fabric was prepared by the method involving the dry heat treatment of a fiber assembly after spinning in the same way as in Example 1. The nonwoven fabric was not subjected to polymer coating treatment. The rebound strength per unit basis weight of this filter element was 0.28 (N·m$^2$/g).

A filter was prepared in the same way as in Example 1. As a result, the rate of change in airflow pressure drop before and after steam sterilization was 1.6%. As a result of conducting the blood test, the leukocyte residual rate was 16.5 x 10$^{-3}$, and the filtration time was 44 minutes, demonstrating that this filter element was practically unsuitable due to low leukocyte removal performance and a long filtration time.

[Comparative Example 8]

The nonwoven fabric used was made of PBT fibers and had a basis weight of 22 g/m$^2$, a thickness of 0.13 mm, a filling rate of 0.12, an average fiber diameter of 1.0 μm, a WC value of 1.7 (gf·cm/cm$^2$), and an area contraction rate of 1.5%. The nonwoven fabric was prepared by the method involving the dry heat treatment of a fiber assembly after spinning in the same way as in Example 1. The nonwoven fabric was not subjected to polymer coating treatment. The rebound strength per unit basis weight of this filter element was 0.28 (N·m$^2$/g).

A filter was prepared in the same way as in Example 1. As a result, the rate of change in airflow pressure drop before and after steam sterilization was 1.4%. As a result of conducting the blood test, the leukocyte residual rate was 15.5 × 10$^{-3}$, and the filtration time was 41 minutes, demonstrating that this filter element was practically unsuitable due to low leukocyte removal performance and a long filtration time.

In the examples described below, filter elements having almost the same rebound strength as that of the corresponding Example (Example 2 or 10) or Comparative Example (Comparative Examples 1 to 9), but differing largely in the area contraction rate of the nonwoven fabric contained in each filter element and the rate of change in airflow pressure drop of a filter before and after exposure to steam of 115°C for 240 minutes were provided to confirm the influence of these two factors.

[Example 17]

As a variation of Example 2, the nonwoven fabric used was made of PET fibers and had a basis weight of 22 g/m$^2$, a thickness of 0.13 mm, a filling rate of 0.12, an average fiber diameter of 1.0 $\mu$m, a WC value of 1.3 (gf·cm/cm$^2$), and an area contraction rate of 10.5%. The nonwoven fabric was prepared by the method involving the dry heat treatment of a fiber assembly after spinning in the same way as in Example 1. Polymer coating treatment was performed in the same way as in Example 1 to form a coat layer covering the PET fibers. The CWST value after the polymer coating treatment was 100 dyn/cm.

A set of 64 stacked sheets of the nonwoven fabric thus polymer-coated was used as a blood processing filter element. The rebound strength per unit basis weight of this filter element was 0.43 (N·m$^2$/g).

A filter was prepared in the same way as in Example 1. As a result, the rate of change in airflow pressure drop before and after steam sterilization was -0.3%. As a result of conducting the blood test, the leukocyte residual rate was $0.7 \times 10^{-3}$, and the filtration time was 20 minutes, demonstrating low blood process pressure and high leukocyte removal performance.

[Example 18]

As a variation of Example 2, the nonwoven fabric used was made of PET fibers and had a basis weight of 22 g/m$^2$, a thickness of 0.13 mm, a filling rate of 0.12, an average fiber diameter of 1.0 $\mu$m, a WC value of 1.4 (gf·cm/cm$^2$), and an area contraction rate of 0.5%. The nonwoven fabric was prepared by the method involving the dry heat treatment of a fiber assembly after spinning in the same way as in Example 1. Polymer coating treatment was performed in the same way as in Example 1 to form a coat layer covering the PET fibers. The CWST value after the polymer coating treatment was 100 dyn/cm.

The nonwoven fabric thus polymer-coated was used as a blood processing filter element. The rebound strength per unit basis weight of this filter element was 0.45 (N·m$^2$/g).

A filter was prepared in the same way as in Example 1. As a result, the rate of change in airflow pressure drop before and after steam sterilization was 0.2%. As a result of conducting the blood test, the leukocyte residual rate was $0.4 \times 10^{-3}$, and the filtration time was 19 minutes, demonstrating low blood process pressure and high leukocyte removal performance.

[Example 19]

As a variation of Example 2, the nonwoven fabric used was made of PET fibers and had a basis weight of 22 g/m$^2$, a thickness of 0.13 mm, a filling rate of 0.12, an average fiber diameter of 1.0 $\mu$m, a WC value of 1.8 (gf·cm/cm$^2$), and an area contraction rate of 11.2%. The nonwoven fabric was prepared by the method involving the dry heat treatment of a fiber assembly after spinning in the same way as in Example 1. Polymer coating treatment was performed in the same way as in Example 1 to form a coat layer covering the PET fibers. The CWST value after the polymer coating treatment was 100 dyn/cm. The nonwoven fabric thus polymer-coated was used as a blood processing filter element. The rebound strength per unit basis weight of this filter element was 0.38 (N·m$^2$/g).

A filter was prepared in the same way as in Example 1. As a result, the rate of change in airflow pressure drop before and after steam sterilization was -1.8%. As a result of conducting the blood test, the leukocyte residual rate was $5.2 \times 10^{-3}$, and the filtration time was 19 minutes, demonstrating low blood process pressure and high leukocyte removal performance.

[Example 20]

As a variation of Example 2, the nonwoven fabric used was made of PET fibers and had a basis weight of 22 g/m$^2$, a thickness of 0.13 mm, a filling rate of 0.12, an average fiber diameter of 1.0 $\mu$m, a WC value of 1.6 (gf·cm/cm$^2$), and an area contraction rate of 9.6%. The nonwoven fabric was prepared by the method involving the dry heat treatment of a fiber assembly after spinning in the same way as in Example 1. Polymer coating treatment was performed in the same way as in Example 1 to form a coat layer covering the PET fibers. The CWST value after the polymer coating treatment was 100 dyn/cm. The nonwoven fabric thus polymer-coated was used as a blood processing filter element. The rebound strength per unit basis weight of this filter element was 0.41 (N·m$^2$/g).

A filter was prepared in the same way as in Example 1. As a result, the rate of change in airflow pressure drop before and after steam sterilization was -1.7%. As a result of conducting the blood test, the leukocyte residual rate was $4.2 \times 10^{-3}$, and the filtration time was 16 minutes, demonstrating low blood process pressure and high leukocyte removal performance.

[Example 21]

As a variation of Example 2, the nonwoven fabric used was made of PET fibers and had a basis weight of 22 g/m$^2$, a thickness of 0.13 mm, a filling rate of 0.12, an average fiber diameter of 1.0 $\mu$m, a WC value of 1.3 (gf·cm/cm$^2$), and an area contraction rate of 10.5%. The nonwoven fabric was prepared by the method involving the dry heat treatment of a fiber assembly after spinning in the same way as in Example 1. The nonwoven fabric was not subjected to polymer coating treatment. The rebound strength per unit basis weight of this filter element was 0.43 (N·m$^2$/g).

A filter was prepared in the same way as in Example 1. As a result, the rate of change in airflow pressure drop before and after steam sterilization was -0.3%. As a result of conducting the blood test, the leukocyte residual rate was $3.3 \times 10^{-3}$, and the filtration time was 22 minutes, demonstrating low blood process pressure and high leukocyte removal performance.

[Example 22]

As a variation of Example 2, the nonwoven fabric used was made of PET fibers and had a basis weight of 22 g/m$^2$, a thickness of 0.13 mm, a filling rate of 0.12, an average fiber diameter of 1.0 $\mu$m, a WC value of 1.4 (gf·cm/cm$^2$), and an area contraction rate of 0.5%. The nonwoven fabric was prepared by the method involving the dry heat treatment of a fiber assembly after spinning in the same way as in Example 1. The nonwoven fabric was not subjected to polymer coating treatment. The rebound strength per unit basis weight of this filter element was 0.45 (N·m$^2$/g).

A filter was prepared in the same way as in Example 1. As a result, the rate of change in airflow pressure drop before and after steam sterilization was 0.2%. As a result of conducting the blood test, the leukocyte residual rate was $2.9 \times 10^{-3}$, and the filtration time was 21 minutes, demonstrating low blood process pressure and high leukocyte removal performance.

[Example 23]

As a variation of Example 2, the nonwoven fabric used was made of PET fibers and had a basis weight of 22 g/m$^2$, a thickness of 0.13 mm, a filling rate of 0.12, an average fiber diameter of 1.0 $\mu$m, a WC value of 1.8 (gf·cm/cm$^2$), and an area contraction rate of 11.2%. The nonwoven fabric was prepared by the method involving the dry heat treatment of a fiber assembly after spinning in the same way as in Example 1. The nonwoven fabric was not subjected to polymer coating treatment. The rebound strength per unit basis weight of this filter element was 0.38 (N·m$^2$/g).

A filter was prepared in the same way as in Example 1. As a result, the rate of change in airflow pressure drop before and after steam sterilization was -1.8%. As a result of conducting the blood test, the leukocyte residual rate was $8.1 \times 10^{-3}$, and the filtration time was 22 minutes, demonstrating low blood process pressure and high leukocyte removal performance.

[Example 24]

As a variation of Example 2, the nonwoven fabric used was made of PET fibers and had a basis weight of 22 g/m$^2$, a thickness of 0.13 mm, a filling rate of 0.12, an average fiber diameter of 1.0 $\mu$m, a WC value of 1.6 (gf·cm/cm$^2$), and an area contraction rate of 9.6%. The nonwoven fabric was prepared by the method involving the dry heat treatment of a fiber assembly after spinning in the same way as in Example 1. The nonwoven fabric was not subjected to polymer coating treatment. The rebound strength per unit basis weight of this filter element was 0.41 (N·m$^2$/g).

A filter was prepared in the same way as in Example 1. As a result, the rate of change in airflow pressure drop before and after steam sterilization was -1.7%. As a result of conducting the blood test, the leukocyte residual rate was $7.3 \times 10^{-3}$, and the filtration time was 20 minutes, demonstrating low blood process pressure and high leukocyte removal performance.

[Example 25]

As a variation of Example 10, the nonwoven fabric used was made of PBT fibers and had a basis weight of 22 g/m$^2$, a thickness of 0.13 mm, a filling rate of 0.12, an average fiber diameter of 1.0 $\mu$m, a WC value of 1.2 (gf·cm/cm$^2$), and an area contraction rate of 10.1%. The nonwoven fabric was prepared by the method involving the dry heat treatment of a fiber assembly after spinning in the same way as in Example 1. Polymer coating treatment was performed in the same way as in Example 1 to form a coat layer covering the PBT fibers. The CWST value after the polymer coating treatment was 100 dyn/cm. The nonwoven fabric thus polymer-coated was used as a blood processing filter element. The rebound strength per unit basis weight of this filter element was 0.45 (N·m$^2$/g).

A filter was prepared in the same way as in Example 1. As a result, the rate of change in airflow pressure drop before and after steam sterilization was 0.2%. As a result of conducting the blood test, the leukocyte residual rate was 0.5 × 10$^{-3}$, and the filtration time was 21 minutes, demonstrating low blood process pressure and high leukocyte removal performance.

[Example 26]

As a variation of Example 10, the nonwoven fabric used was made of PBT fibers and had a basis weight of 22 g/m$^2$, a thickness of 0.13 mm, a filling rate of 0.12, an average fiber diameter of 1.0 μm, a WC value of 1.3 (gf·cm/cm$^2$), and an area contraction rate of 0.4%. The nonwoven fabric was prepared by the method involving the dry heat treatment of a fiber assembly after spinning in the same way as in Example 1. Polymer coating treatment was performed in the same way as in Example 1 to form a coat layer covering the PBT fibers. The CWST value after the polymer coating treatment was 100 dyn/cm. The nonwoven fabric thus polymer-coated was used as a blood processing filter element. The rebound strength per unit basis weight of this filter element was 0.47 (N·m$^2$/g).

A filter was prepared in the same way as in Example 1. As a result, the rate of change in airflow pressure drop before and after steam sterilization was -0.1%. As a result of conducting the blood test, the leukocyte residual rate was 0.1 × 10$^{-3}$, and the filtration time was 18 minutes, demonstrating low blood process pressure and high leukocyte removal performance.

[Example 27]

As a variation of Example 10, the nonwoven fabric used was made of PBT fibers and had a basis weight of 22 g/m$^2$, a thickness of 0.13 mm, a filling rate of 0.12, an average fiber diameter of 1.0 μm, a WC value of 1.6 (gf·cm/cm$^2$), and an area contraction rate of 10.9%. The nonwoven fabric was prepared by the method involving the dry heat treatment of a fiber assembly after spinning in the same way as in Example 1. Polymer coating treatment was performed in the same way as in Example 1 to form a coat layer covering the PBT fibers. The CWST value after the polymer coating treatment was 100 dyn/cm. The nonwoven fabric thus polymer-coated was used as a blood processing filter element. The rebound strength per unit basis weight of this filter element was 0.40 (N·m$^2$/g).

A filter was prepared in the same way as in Example 1. As a result, the rate of change in airflow pressure drop before and after steam sterilization was -1.7%. As a result of conducting the blood test, the leukocyte residual rate was 4.2 × 10$^{-3}$, and the filtration time was 19 minutes, demonstrating low blood process pressure and high leukocyte removal performance.

[Example 28]

As a variation of Example 10, the nonwoven fabric used was made of PBT fibers and had a basis weight of 22 g/m$^2$, a thickness of 0.13 mm, a filling rate of 0.12, an average fiber diameter of 1.0 μm, a WC value of 1.4 (gf·cm/cm$^2$), and an area contraction rate of 9.2%. The nonwoven fabric was prepared by the method involving the dry heat treatment of a fiber assembly after spinning in the same way as in Example 1. Polymer coating treatment was performed in the same way as in Example 1 to form a coat layer covering the PBT fibers. The CWST value after the polymer coating treatment was 100 dyn/cm. The nonwoven fabric thus polymer-coated was used as a blood processing filter element. The rebound strength per unit basis weight of this filter element was 0.43 (N·m$^2$/g).

A filter was prepared in the same way as in Example 1. As a result, the rate of change in airflow pressure drop before and after steam sterilization was -1.6%. As a result of conducting the blood test, the leukocyte residual rate was 3.8 × 10$^{-3}$, and the filtration time was 17 minutes, demonstrating low blood process pressure and high leukocyte removal performance.

[Example 29]

As a variation of Example 10, the nonwoven fabric used was made of PBT fibers and had a basis weight of 22 g/m$^2$, a thickness of 0.13 mm, a filling rate of 0.12, an average fiber diameter of 1.0 μm, a WC value of 1.2 (gf·cm/cm$^2$), and an area contraction rate of 10.1%. The nonwoven fabric was prepared by the method involving the dry heat treatment of a fiber assembly after spinning in the same way as in Example 1. The nonwoven fabric was not subjected to polymer coating treatment. The rebound strength per unit basis weight of this filter element was 0.45 (N·m$^2$/g).

A filter was prepared in the same way as in Example 1. As a result, the rate of change in airflow pressure drop before and after steam sterilization was 0.2%. As a result of conducting the blood test, the leukocyte residual rate was $2.5 \times 10^{-3}$, and the filtration time was 28 minutes, demonstrating low blood process pressure and high leukocyte removal performance.

[Example 30]

As a variation of Example 10, the nonwoven fabric used was made of PBT fibers and had a basis weight of 22 $g/m^2$, a thickness of 0.13 mm, a filling rate of 0.12, an average fiber diameter of 1.0 $\mu$m, a WC value of 1.3 (gf·cm/cm$^2$), and an area contraction rate of 0.4%. The nonwoven fabric was prepared by the method involving the dry heat treatment of a fiber assembly after spinning in the same way as in Example 1. The nonwoven fabric was not subjected to polymer coating treatment. The rebound strength per unit basis weight of this filter element was 0.47 (N·m$^2$/g).
A filter was prepared in the same way as in Example 1. As a result, the rate of change in airflow pressure drop before and after steam sterilization was -0.1%. As a result of conducting the blood test, the leukocyte residual rate was $2.1 \times 10^{-3}$, and the filtration time was 29 minutes, demonstrating low blood process pressure and high leukocyte removal performance.

[Example 31]

As a variation of Example 10, the nonwoven fabric used was made of PBT fibers and had a basis weight of 22 $g/m^2$, a thickness of 0.13 mm, a filling rate of 0.12, an average fiber diameter of 1.0 $\mu$m, a WC value of 1.6 (gf·cm/cm$^2$), and an area contraction rate of 10.9%. The nonwoven fabric was prepared by the method involving the dry heat treatment of a fiber assembly after spinning in the same way as in Example 1. The nonwoven fabric was not subjected to polymer coating treatment. The rebound strength per unit basis weight of this filter element was 0.40 (N·m$^2$/g).
A filter was prepared in the same way as in Example 1. As a result, the rate of change in airflow pressure drop before and after steam sterilization was -1.7%. As a result of conducting the blood test, the leukocyte residual rate was $7.4 \times 10^{-3}$, and the filtration time was 28 minutes, demonstrating low blood process pressure and high leukocyte removal performance.

[Example 32]

As a variation of Example 10, the nonwoven fabric used was made of PBT fibers and had a basis weight of 22 $g/m^2$, a thickness of 0.13 mm, a filling rate of 0.12, an average fiber diameter of 1.0 $\mu$m, a WC value of 1.4 (gf·cm/cm$^2$), and an area contraction rate of 9.2%. The nonwoven fabric was prepared by the method involving the dry heat treatment of a fiber assembly after spinning in the same way as in Example 1. The nonwoven fabric was not subjected to polymer coating treatment. The rebound strength per unit basis weight of this filter element was 0.43 (N·m$^2$/g).
A filter was prepared in the same way as in Example 1. As a result, the rate of change in airflow pressure drop before and after steam sterilization was -1.6%. As a result of conducting the blood test, the leukocyte residual rate was $6.6 \times 10^{-3}$, and the filtration time was 28 minutes, demonstrating low blood process pressure and high leukocyte removal performance.

[Comparative Example 9]

As a variation of Comparative Example 1, the nonwoven fabric used was made of PET fibers and had a basis weight of 22 g/m$^2$, a thickness of 0.13 mm, a filling rate of 0.12, an average fiber diameter of 1.0 $\mu$m, a WC value of 2.4 (gf·cm/cm$^2$), and an area contraction rate of 10.8%. The nonwoven fabric was prepared by the method involving the dry heat treatment of a fiber assembly after spinning in the same way as in Example 1.
Polymer coating treatment was performed in the same way as in Example 1 to form a coat layer covering the PET fibers. The CWST value after the polymer coating treatment was 100 dyn/cm. The nonwoven fabric thus polymer-coated was used as a blood processing filter element. The rebound strength per unit basis weight of this filter element was 0.27 (N·m$^2$/g).
A filter was prepared in the same way as in Example 1. As a result, the rate of change in airflow pressure drop before and after steam sterilization was 2.5%. As a result of conducting the blood test, the leukocyte residual rate was $12.5 \times 10^{-3}$, and the filtration time was 22 minutes, demonstrating that this filter element was practically unsuitable due to low leukocyte removal performance, though its filtration time was short.

[Comparative Example 10]

As a variation of Comparative Example 2, the nonwoven fabric used was made of PET fibers and had a basis weight of 22 g/m$^2$, a thickness of 0.13 mm, a filling rate of 0.12, an average fiber diameter of 1.0 $\mu$m, a WC value of 1.4 (gf·cm/cm$^2$), and an area contraction rate of 0.7%. The nonwoven fabric was prepared by the method involving the dry heat treatment of a fiber assembly after spinning in the same way as in Example 1. Polymer coating treatment was performed in the same way as in Example 1 to form a coat layer covering the PET fibers. The CWST value after the polymer coating treatment was 100 dyn/cm. The nonwoven fabric thus polymer-coated was used as a blood processing filter element. The rebound strength per unit basis weight of this filter element was 0.28 (N·m$^2$/g).
A filter was prepared in the same way as in Example 1. As a result, the rate of change in airflow pressure drop before and after steam sterilization was 2.4%. As a result of conducting the blood test, the leukocyte residual rate was 11.1 $\times$ 10$^{-3}$, and the filtration time was 21 minutes, demonstrating that this filter element was practically unsuitable due to low leukocyte removal performance, though its filtration time was short.

[Comparative Example 11]

As a variation of Comparative Example 3, the nonwoven fabric used was made of PET fibers and had a basis weight of 22 g/m$^2$, a thickness of 0.13 mm, a filling rate of 0.12, an average fiber diameter of 1.0 $\mu$m, a WC value of 2.4 (gf·cm/cm$^2$), and an area contraction rate of 10.8%. The nonwoven fabric was prepared by the method involving the dry heat treatment of a fiber assembly after spinning in the same way as in Example 1. The nonwoven fabric was not subjected to polymer coating treatment. The rebound strength per unit basis weight of this filter element was 0.27 (N·m$^2$/g).
A filter was prepared in the same way as in Example 1. As a result, the rate of change in airflow pressure drop before and after steam sterilization was 2.5%. As a result of conducting the blood test, the leukocyte residual rate was 16.4 $\times$ 10$^{-3}$, and the filtration time was 30 minutes, demonstrating that this filter element was practically unsuitable due to low leukocyte removal performance, though its filtration time was acceptable.

[Comparative Example 12]

As a variation of Comparative Example 4, the nonwoven fabric used was made of PET fibers and had a basis weight of 22 g/m$^2$, a thickness of 0.13 mm, a filling rate of 0.12, an average fiber diameter of 1.0 $\mu$m, a WC value of 1.4 (gf·cm/cm$^2$), and an area contraction rate of 0.7%. The nonwoven fabric was prepared by the method involving the dry heat treatment of a fiber assembly after spinning in the same way as in Example 1. The nonwoven fabric was not subjected to polymer coating treatment. The rebound strength per unit basis weight of this filter element was 0.28 (N·m$^2$/g).
A filter was prepared in the same way as in Example 1. As a result, the rate of change in airflow pressure drop before and after steam sterilization was 2.4%. As a result of conducting the blood test, the leukocyte residual rate was 15.3 $\times$ 10$^{-3}$, and the filtration time was 30 minutes, demonstrating that this filter element was practically unsuitable due to low leukocyte removal performance, though its filtration time was acceptable.

[Comparative Example 13]

As a variation of Comparative Example 5, the nonwoven fabric used was made of PBT fibers and had a basis weight of 22 g/m$^2$, a thickness of 0.13 mm, a filling rate of 0.12, an average fiber diameter of 1.0 $\mu$m, a WC value of 2.3 (gf·cm/cm$^2$), and an area contraction rate of 10.6%. The nonwoven fabric was prepared by the method involving the dry heat treatment of a fiber assembly after spinning in the same way as in Example 1. Polymer coating treatment was performed in the same way as in Example 1 to form a coat layer covering the PBT fibers. The CWST value after the polymer coating treatment was 100 dyn/cm. The nonwoven fabric thus polymer-coated was used as a blood processing filter element. The rebound strength per unit basis weight of this filter element was 0.28 (N·m$^2$/g).
A filter was prepared in the same way as in Example 1. As a result, the rate of change in airflow pressure drop before and after steam sterilization was 2.4%. As a result of conducting the blood test, the leukocyte residual rate was 11.2 $\times$ 10$^{-3}$, and the filtration time was 20 minutes, demonstrating that this filter element was practically unsuitable due to low leukocyte removal performance, though its filtration time was short.

[Comparative Example 14]

As a variation of Comparative Example 6, the nonwoven fabric used was made of PBT fibers and had a basis weight of 22 g/m$^2$, a thickness of 0.13 mm, a filling rate of 0.12, an average fiber diameter of 1.0 $\mu$m, a WC value of 1.3 (gf·cm/cm$^2$), and an area contraction rate of 0.5%. The nonwoven fabric was prepared by the method involving the dry heat treatment of a fiber assembly after spinning in the same way as in Example 1. Polymer coating treatment was performed in the same way as in Example 1 to form a coat layer covering the PBT fibers. The CWST value after the polymer coating treatment was 100 dyn/cm. The nonwoven fabric thus polymer-coated was used as a blood processing filter element. The rebound strength per unit basis weight of this filter element was 0.29 (N·m$^2$/g).

A filter was prepared in the same way as in Example 1. As a result, the rate of change in airflow pressure drop before and after steam sterilization was 2.2%. As a result of conducting the blood test, the leukocyte residual rate was $10.3 \times 10^{-3}$, and the filtration time was 21 minutes, demonstrating that this filter element was practically unsuitable due to low leukocyte removal performance, though its filtration time was short.

[Comparative Example 15]

As a variation of Comparative Example 7, the nonwoven fabric used was made of PBT fibers and had a basis weight of 22 g/m$^2$, a thickness of 0.13 mm, a filling rate of 0.12, an average fiber diameter of 1.0 $\mu$m, a WC value of 2.3 (gf·cm/cm$^2$), and an area contraction rate of 10.6%. The nonwoven fabric was prepared by the method involving the dry heat treatment of a fiber assembly after spinning in the same way as in Example 1. The nonwoven fabric was not subjected to polymer coating treatment. The rebound strength per unit basis weight of this filter element was 0.28 (N·m$^2$/g).

A filter was prepared in the same way as in Example 1. As a result, the rate of change in airflow pressure drop before and after steam sterilization was 2.4%. As a result of conducting the blood test, the leukocyte residual rate was $14.5 \times 10^{-3}$, and the filtration time was 42 minutes, demonstrating that this filter element was practically unsuitable due to low leukocyte removal performance and a long filtration time.

[Comparative Example 16]

As a variation of Comparative Example 8, the nonwoven fabric used was made of PBT fibers and had a basis weight of 22 g/m$^2$, a thickness of 0.13 mm, a filling rate of 0.12, an average fiber diameter of 1.0 $\mu$m, a WC value of 1.3 (gf·cm/cm$^2$), and an area contraction rate of 0.5%. The nonwoven fabric was prepared by the method involving the dry heat treatment of a fiber assembly after spinning in the same way as in Example 1. The nonwoven fabric was not subjected to polymer coating treatment. The rebound strength per unit basis weight of this filter element was 0.29 (N·m$^2$/g).

A filter was prepared in the same way as in Example 1. As a result, the rate of change in airflow pressure drop before and after steam sterilization was 2.2%. As a result of conducting the blood test, the leukocyte residual rate was $13.5 \times 10^{-3}$, and the filtration time was 40 minutes, demonstrating that this filter element was practically unsuitable due to low leukocyte removal performance and a long filtration time.

The blood evaluation results of Examples 1 to 32 and Comparative Examples 1 to 16 are shown in Tables 1 to 6.

[Table 1]

| | Example 1 | Example 2 | Example 3 | Example 4 | Example 5 | Example 6 | Example 7 | Example 8 |
|---|---|---|---|---|---|---|---|---|
| Nonwoven fabric filter material | PET | PET | PET | PET | PET | PET | PET | PET |
| Basis weight (g/m$^2$) | 22 | 22 | 22 | 22 | 22 | 22 | 22 | 22 |
| Thickness (mm) | 0.13 | 0.13 | 0.13 | 0.13 | 0.13 | 0.13 | 0.13 | 0.13 |
| Filling rate | 0.12 | 0.12 | 0.12 | 0.12 | 0.12 | 0.12 | 0.12 | 0.12 |
| Rebound strength per unit basis weight (N·m2/g) | 0.40 | 0.40 | 0.31 | 0.31 | 0.40 | 0.40 | 0.31 | 0.31 |
| WC (gf·cm/cm$^2$) | 2.1 | 1.7 | 2.1 | 1.7 | 2.1 | 1.7 | 2.1 | 1.7 |

(continued)

| | Example 1 | Example 2 | Example 3 | Example 4 | Example 5 | Example 6 | Example 7 | Example 8 |
|---|---|---|---|---|---|---|---|---|
| Rate of change in airflow pressure drop (%) | 1.8 | 1.6 | 2.0 | 1.9 | 1.8 | 1.6 | 2.0 | 1.9 |
| Area contraction rate (%) | 1.3 | 1.1 | 1.5 | 1.4 | 1.3 | 1.1 | 1.5 | 1.4 |
| Presence or absence of coating treatment | Present | Present | Present | Present | Absent | Absent | Absent | Absent |
| Leukocyte residual rate ($\times 10^{-3}$) | 0.7 | 0.4 | 5.2 | 4.1 | 3.3 | 2.8 | 8.3 | 7.3 |
| Filtration time (min) | 19 | 17 | 19 | 18 | 22 | 21 | 23 | 20 |

[Table 2]

| | Example 9 | Example 10 | Example 11 | Example 12 | Example 13 | Example 14 | Example 15 | Example 16 |
|---|---|---|---|---|---|---|---|---|
| Nonwoven fabric filter material | PBT | PBT | PBT | PBT | PBT | PBT | PBT | PBT |
| Basis weight (g/m$^2$) | 22 | 22 | 22 | 22 | 22 | 22 | 22 | 22 |
| Thickness (mm) | 0.13 | 0.13 | 0.13 | 0.13 | 0.13 | 0.13 | 0.13 | 0.13 |
| Filling rate | 0.12 | 0.12 | 0.12 | 0.12 | 0.12 | 0.12 | 0.12 | 0.12 |
| Rebound strength per unit basis weight (N·m2/g) | 0.40 | 0.40 | 0.31 | 0.31 | 0.40 | 0.40 | 0.31 | 0.31 |
| WC (gf·cm/cm$^2$) | 2.1 | 1.7 | 2.1 | 1.7 | 2.1 | 1.7 | 2.1 | 1.7 |
| Rate of change in airflow pressure drop (%) | 1.7 | 1.5 | 1.9 | 1.8 | 1.7 | 1.5 | 1.9 | 1.8 |
| Area contraction rate (%) | 1.2 | 1.0 | 1.4 | 1.3 | 1.2 | 1.0 | 1.4 | 1.3 |

(continued)

|  | Example 9 | Example 10 | Example 11 | Example 12 | Example 13 | Example 14 | Example 15 | Example 16 |
|---|---|---|---|---|---|---|---|---|
| Presence or absence of coating treatment | Present | Present | Present | Present | Absent | Absent | Absent | Absent |
| Leukocyte residual rate ($\times 10^{-3}$) | 0.5 | 0.1 | 4.5 | 3.8 | 2.8 | 2.4 | 7.9 | 6.8 |
| Filtration time (min) | 19 | 18 | 19 | 20 | 29 | 28 | 29 | 29 |

[Table 3]

|  | Example 17 | Example 18 | Example 19 | Example 20 | Example 21 | Example 22 | Example 23 | Example 24 |
|---|---|---|---|---|---|---|---|---|
| Nonwoven fabric filter material | PET | PET | PET | PET | PET | PET | PET | PET |
| Basis weight (g/m$^2$) | 22 | 22 | 22 | 22 | 22 | 22 | 22 | 22 |
| Thickness (mm) | 0.13 | 0.13 | 0.13 | 0.13 | 0.13 | 0.13 | 0.13 | 0.13 |
| Filling rate | 0.12 | 0.12 | 0.12 | 0.12 | 0.12 | 0.12 | 0.12 | 0.12 |
| Rebound strength per unit basis weight (N·m2/g) | 0.43 | 0.45 | 0.38 | 0.41 | 0.43 | 0.45 | 0.38 | 0.41 |
| WC (gf·cm/cm$^2$) | 1.3 | 1.4 | 1.8 | 1.6 | 1.3 | 1.4 | 1.8 | 1.6 |
| Rate of change in airflow pressure drop (%) | -0.3 | 0.2 | -1.8 | -1.7 | -0.3 | 0.2 | -1.8 | -1.7 |
| Area contraction rate (%) | 10.5 | 0.5 | 11.2 | 9.6 | 10.5 | 0.5 | 11.2 | 9.6 |
| Presence or absence of coating treatment | Present | Present | Present | Present | Absent | Absent | Absent | Absent |
| Leukocyte residual rate ($\times 10^{-3}$) | 0.7 | 0.4 | 5.2 | 4.2 | 3.3 | 2.9 | 8.1 | 7.3 |
| Filtration time (min) | 20 | 19 | 19 | 16 | 22 | 21 | 22 | 20 |

[Table 4]

| | Example 25 | Example 26 | Example 27 | Example 28 | Example 29 | Example 30 | Example 31 | Example 32 |
|---|---|---|---|---|---|---|---|---|
| Nonwoven fabric filter material | PBT | PBT | PBT | PBT | PBT | PBT | PBT | PBT |
| Basis weight (g/m$^2$) | 22 | 22 | 22 | 22 | 22 | 22 | 22 | 22 |
| Thickness (mm) | 0.13 | 0.13 | 0.13 | 0.13 | 0.13 | 0.13 | 0.13 | 0.13 |
| Filling rate | 0.12 | 0.12 | 0.12 | 0.12 | 0.12 | 0.12 | 0.12 | 0.12 |
| Rebound strength per unit basis weight (N·m2/g) | 0.45 | 0.47 | 0.40 | 0.43 | 0.45 | 0.47 | 0.40 | 0.43 |
| WC (gf·cm/cm$^2$) | 1.2 | 1.3 | 1.6 | 1.4 | 1.2 | 1.3 | 1.6 | 1.4 |
| Rate of change in airflow pressure drop (%) | 0.2 | -0.1 | -1.7 | -1.6 | 0.2 | -0.1 | -1.7 | -1.6 |
| Area contraction rate (%) | 10.1 | 0.4 | 10.9 | 9.2 | 10.1 | 0.4 | 10.9 | 9.2 |
| Presence or absence of coating treatment | Present | Present | Present | Present | Absent | Absent | Absent | Absent |
| Leukocyte residual rate (×10$^{-3}$) | 0.5 | 0.1 | 4.2 | 3.8 | 2.5 | 2.1 | 7.4 | 6.6 |
| Filtration time (min) | 21 | 18 | 19 | 17 | 28 | 29 | 28 | 28 |

[Table 5]

| | Comparative Example 1 | Comparative Example 2 | Comparative Example 3 | Comparative Example 4 | Comparative Example 5 | Comparative Example 6 | Comparative Example 7 | Comparative Example 8 |
|---|---|---|---|---|---|---|---|---|
| Nonwoven fabric filter material | PET | PET | PET | PET | PBT | PBT | PBT | PBT |
| Basis weight (g/m$^2$) | 22 | 22 | 22 | 22 | 22 | 22 | 22 | 22 |
| Thickness (mm) | 0.13 | 0.13 | 0.13 | 0.13 | 0.13 | 0.13 | 0.13 | 0.13 |
| Filling rate | 0.12 | 0.12 | 0.12 | 0.12 | 0.12 | 0.12 | 0.12 | 0.12 |
| Rebound strength per unit basis weight (N·m2/g) | 0.28 | 0.28 | 0.28 | 0.28 | 0.28 | 0.28 | 0.28 | 0.28 |
| WC (gf·cm/cm$^2$) | 2.1 | 1.7 | 2.1 | 1.7 | 2.1 | 1.7 | 2.1 | 1.7 |
| Rate of change in airflow pressure drop (%) | 1.7 | 1.6 | 1.7 | 1.6 | 1.6 | 1.4 | 1.6 | 1.4 |
| Area contraction rate (%) | 2.7 | 1.6 | 2.7 | 1.6 | 2.6 | 1.5 | 2.6 | 1.5 |
| Presence or absence of coating treatment | Present | Present | Absent | Absent | Present | Present | Absent | Absent |
| Leukocyte residual rate (×10$^{-3}$) | 16.5 | 13.8 | 19.5 | 17.4 | 13.5 | 12.1 | 16.5 | 15.5 |
| Filtration time (min) | 21 | 20 | 29 | 30 | 22 | 23 | 44 | 41 |

EP 3 311 858 B1

[Table 6]

| | Comparative Example 9 | Comparative Example 10 | Comparative Example 11 | Comparative Example 12 | Comparative Example 13 | Comparative Example 14 | Comparative Example 15 | Comparative Example 16 |
|---|---|---|---|---|---|---|---|---|
| Nonwoven fabric filter material | PET | PET | PET | PET | PBT | PBT | PBT | PBT |
| Basis weight (g/m$^2$) | 22 | 22 | 22 | 22 | 22 | 22 | 22 | 22 |
| Thickness (mm) | 0.13 | 0.13 | 0.13 | 0.13 | 0.13 | 0.13 | 0.13 | 0.13 |
| Filling rate | 0.12 | 0.12 | 0.12 | 0.12 | 0.12 | 0.12 | 0.12 | 0.12 |
| Rebound strength per unit basis weight (N·m2/g) | 0.27 | 0.28 | 0.27 | 0.28 | 0.28 | 0.29 | 0.28 | 0.29 |
| WC (gf·cm/cm$^2$) | 2.4 | 1.4 | 2.4 | 1.4 | 2.3 | 1.3 | 2.3 | 1.3 |
| Rate of change in airflow pressure drop (%) | 2.5 | 2.4 | 2.5 | 2.4 | 2.4 | 2.2 | 2.4 | 2.2 |
| Area contraction rate (%) | 10.8 | 0.7 | 10.8 | 0.7 | 10.6 | 0.5 | 10.6 | 0.5 |
| Presence or absence of coating treatment | Present | Present | Absent | Absent | Present | Present | Absent | Absent |
| Leukocyte residual rate ($\times 10^{-3}$) | 12.5 | 11.1 | 16.4 | 15.3 | 11.2 | 10.3 | 14.5 | 13.5 |
| Filtration time (min) | 22 | 21 | 30 | 30 | 20 | 21 | 42 | 40 |

EP 3 311 858 B1

As shown in Tables 1, 2, and 5, it was able to be confirmed from the results of Examples 1 to 16 and Comparative Examples 1 to 8 that leukocyte removal performance and a short filtration time, i.e., favorable flowability, can be achieved by producing a blood processing filter using a nonwoven fabric having rebound strength of 0.30 (N·m$^2$/g) per unit basis weight. It was able to be further confirmed that the leukocyte removal performance can be further improved by setting the WC value of the nonwoven fabric to be low. In addition, it was confirmed that the formation of a polymer coat layer on the nonwoven fabric is effective for further improving the leukocyte removal performance and shortening the filtration time and contributes to improvement in performance balance.

As shown in Tables 3, 4, and 6, it was able to be confirmed from the results of Examples 17 to 32 and Comparative Examples 9 to 16 that the leukocyte removal performance can be further improved by setting the rate of change in airflow pressure drop of the filter to be low while maintaining the high rebound strength per unit basis weight of the filter element. In addition, it was also confirmed that the additional effects of further enhancing the leukocyte removal performance and shortening the filtration time are obtained by setting the area contraction rate of the nonwoven fabric constituting the filter element to be low.

When PET and PBT were compared as a material for the nonwoven fabric, the PBT nonwoven fabric was found to significantly extend the filtration time in the absence of coating treatment as compared with in the presence of coating treatment, whereas the influence of the presence or absence of coating treatment on the filtration time was found to be smaller in the PET nonwoven fabric than in PBT. This suggests that the PET nonwoven fabric permits filter design without coating treatment and is effective for reducing production cost, when the leukocyte removal performance can sufficiently satisfy the standard (Examples 5, 6, 21, and 22).

Industrial Applicability

The blood processing filter element of the present invention and the blood processing filter using this blood processing filter element can be used for removing unnecessary components (e.g., aggregates, pathogenic substances (viruses, bacteria, protozoa, infected red cells, etc.), and drugs for blood processing) contained in blood.

Particularly, the blood processing filter of the present invention has higher leukocyte removal performance and can shorten a processing time without causing clogging, as compared with conventional ones. Therefore, the blood processing filter of the present invention can be suitably used as a leukocyte removal filter for capturing leukocytes.

Reference Signs List

1 ... Rigid container, 3 ... First port (liquid inlet/outlet), 3a ... Inlet-side port, 4 ... Second port (liquid inlet/outlet), 4a ... Outlet-side port, 5 ... Filter element, 6 ... Outer peripheral welded part, 7 ... Space on the first port side, 8 ... Space on the second port side, 9 ... Outer edge of the filter element, 10 ... Blood processing filter, 50 ... Blood processing filter, 51 ... Filter element, 53 ... Flexible container, 55 ... bonded part, 56 ... Protruding part of nonwoven fabric.

**Claims**

1. A blood processing filter (10) comprising:

   a container (1) housing a filter element (5) for blood processing, wherein the container (1) comprises an inlet-side container member, and an outlet-side container member, wherein the filter element (5) is held by being sandwiched between the inlet-side container member and the outlet side container member and an inside of the blood processing filter is partitioned by the filter element into an inlet-side space (7) and an outlet-side space (8) and
   wherein the filter element (5) comprises a nonwoven polymer fabric being melt-blown and being further heat-treated using a heat source having a temperature equal to or higher than [melting point of the polymer - 120]°C during a heating-time of at least 3 seconds or longer, wherein the rebound strength (N) per unit basis weight (g/m$^2$) before steam heat treatment is 0.3 N.m$^2$/g or larger and wherein the rebound strength after the steam heat treatment is larger than the rebound strength before the steam heat treatment.

2. A blood processing filter (50) comprising:a flexible container (53) housing a filter element (51) for blood processing, wherein the container (53) has an inlet (3) and an outlet (4), wherein the filter element (51) is held by being bonded with the flexible container (53), and the inside of the blood processing filter (50) is partitioned by the filter element (51) into an inlet-side space (7) and an outlet-side space (8) and wherein the filter element (51) comprises a nonwoven polymer fabric being melt-blown and being further heat-treated using a heat source having a temperature equal to or higher than [melting point of the polymer - 120]°C during a heating-time of at least 3 seconds or longer, wherein the rebound strength (N) per unit basis weight ($g/m^2$) before steam heat treatment is 0.3 $N.m^2/g$ or larger and wherein the rebound strength after the steam heat treatment is larger than the rebound strength before the steam heat treatment.

3. The blood processing filter (10, 50) according to claim 1 or 2 wherein the nonwoven polymer fabric has a work of compression (WC) value corresponding to a basis weight of 40 $g/m^2$ of 2.0 $gfcm/cm^2$ )or smaller.

4. The blood processing filter (10, 50) according to any of claims 1 or 3, wherein the area contraction rate of the nonwoven polymer fabric by exposure to steam of 115°C for 240 minutes is 10% or less.

5. The blood processing filter (10, 50) according to any one of claims 1 to 4, wherein the rate of change in airflow pressure drop before and after exposure to steam of 115°C for 240 minutes is $\pm2\%$ or less.

6. A blood processing method comprising the step of performing a treatment of applying centrifugal force to the blood processing filter (50) according to claim 2.


**Patentansprüche**

1. Blutverarbeitungsfilter (10), umfassend:
   einen Behälter (1), der ein Filterelement (5) zur Blutverarbeitung beherbergt, wobei der Behälter (1) ein Behälterelement der Einlassseite und ein Behälterelement der Auslassseite umfasst, wobei das Filterelement (5) dadurch gehalten wird, dass es zwischen dem Behälterelement der Einlassseite und dem Behälterelement der Auslassseite eingeklemmt ist, und das Innere des Blutverarbeitungsfilters durch das Filterelement in einen Raum der Einlassseite (7) und einen Raum der Auslassseite (8) partitioniert ist und wobei das Filterelement (5) einen Polymervliesstoff umfasst, der schmelzgeblasen ist und weiterhin unter Verwendung einer Wärmequelle, die eine Temperatur aufweist, welche größer oder gleich [Schmelzpunkt des Polymers - 120] °C ist, während einer Erwärmungszeit von wenigstens 3 Sekunden oder länger wärmebehandelt ist, wobei die Rückprallfestigkeit (N) pro Einheit der flächenbezogenen Masse ($g/m^2$) vor der Dampfwärmebehandlung 0,3 $N \cdot m^2/g$ oder mehr beträgt und wobei die Rückprallfestigkeit nach der Dampfwärmebehandlung größer ist als die Rückprallfestigkeit vor der Dampfwärmebehandlung.

2. Blutverarbeitungsfilter (50), umfassend: einen flexiblen Behälter (53), der ein Filterelement (51) zur Blutverarbeitung beherbergt, wobei der Behälter (53) einen Einlass (3) und einen Auslass (4) aufweist, wobei das Filterelement (51) dadurch gehalten wird, dass es mit dem flexiblen Behälter (53) verklebt ist, und das Innere des Blutverarbeitungsfilters (50) durch das Filterelement (51) in einen Raum der Einlassseite (7) und einen Raum der Auslassseite (8) partitioniert ist und wobei das Filterelement (51) einen Polymervliesstoff umfasst, der schmelzgeblasen ist und weiterhin unter Verwendung einer Wärmequelle, die eine Temperatur aufweist, welche größer oder gleich [Schmelzpunkt des Polymers - 120] °C ist, während einer Erwärmungszeit von wenigstens 3 Sekunden oder länger wärmebehandelt ist, wobei die Rückprallfestigkeit (N) pro Einheit der flächenbezogenen Masse ($g/m^2$) vor der Dampfwärmebehandlung 0,3 $N \cdot m^2/g$ oder mehr beträgt und wobei die Rückprallfestigkeit nach der Dampfwärmebehandlung größer ist als die Rückprallfestigkeit vor der Dampfwärmebehandlung.

3. Blutverarbeitungsfilter (10, 50) gemäß Anspruch 1 oder 2, wobei der Polymervliesstoff einen Wert der Kompressionsarbeit (WC) aufweist, der einer flächenbezogenen Masse von 40 $g/m^2$ von 2,0 Pond·cm/cm$^2$ oder weniger entspricht.

4. Blutverarbeitungsfilter (10, 50) gemäß einem der Ansprüche 1 oder 3, wobei die Flächenkontraktionsrate des Polymervliesstoffs bei Einwirkung von Dampf von 115 °C während 240 Minuten 10% oder weniger beträgt.

5. Blutverarbeitungsfilter (10, 50) gemäß einem der Ansprüche 1 bis 4, wobei die Rate der Veränderung des Luftstromdruckabfalls vor und nach der Einwirkung von Dampf von 115 °C während 240 Minuten $\pm$ 2% oder weniger beträgt.

6. Blutverarbeitungsverfahren, das den Schritt des Durchführens einer Behandlung unter Anwendung von Zentrifugalkraft auf den Blutverarbeitungsfilter (50) gemäß Anspruch 2 umfasst.

**Revendications**

1. Filtre de traitement du sang (10) comprenant :

un récipient (1) logeant un élément filtrant (5) pour le traitement du sang, dans lequel le récipient (1) comprend un élément formant récipient côté entrée et un élément formant récipient côté sortie, dans lequel l'élément filtrant (5) est maintenu en étant pris en sandwich entre l'élément formant récipient côté entrée et l'élément formant récipient côté sortie et un intérieur du filtre de traitement du sang est séparé par l'élément filtrant en un espace côté entrée (7) et un espace côté sortie (8) et

dans lequel l'élément filtrant (5) comprend un tissu polymère non tissé étant obtenu par fusion-soufflage et étant en outre traité thermiquement au moyen d'une source de chaleur ayant une température supérieure ou égale au point de fusion du polymère, - 120° C, pendant un temps de chauffage d'au moins 3 secondes ou plus, dans lequel la dureté par rebondissement (N) par unité de poids de base $(g/m^2)$ avant traitement thermique à la vapeur est supérieure ou égale à 0,3 $N.m^2/g$ et dans lequel la dureté par rebondissement après le traitement thermique à la vapeur est supérieure à la dureté par rebondissement avant le traitement thermique à la vapeur.

2. Filtre de traitement du sang (50) comprenant : un récipient flexible (53) contenant un élément filtrant (51) pour le traitement du sang, dans lequel le récipient (53) possède une entrée (3) et une sortie (4), dans lequel l'élément filtrant (51) est maintenu en étant assemblé avec le récipient flexible (53), et l'intérieur du filtre de traitement du sang (50) est séparé par l'élément filtrant (51) en un espace côté entrée (7) et un espace côté sortie (8) et dans lequel l'élément filtrant (51) comprend un tissu polymère non tissé étant obtenu par fusion-soufflage et étant en outre traité thermiquement au moyen d'une source de chaleur ayant une température supérieure ou égale au point de fusion du polymère, - 120° C, pendant un temps de chauffage d'au moins 3 secondes ou plus, dans lequel la dureté par rebondissement (N) par unité de poids de base $(g/m^2)$ avant traitement thermique à la vapeur est supérieure ou égale à 0,3 $N.m^2/g$ et dans lequel la dureté par rebondissement après le traitement thermique à la vapeur est supérieure à la dureté par rebondissement avant le traitement thermique à la vapeur.

3. Filtre de traitement du sang (10, 50) selon la revendication 1 ou 2, dans lequel le tissu polymère non tissé a une valeur de travail de compression (WC) correspondant à un poids de base de 40 $g/m^2$ inférieure ou égale à 2,0 gf cm/cm$^2$.

4. Filtre de traitement du sang (10, 50) selon l'une quelconque des revendications 1 ou 3, dans lequel le taux de contraction de zone du tissu polymère non tissé par exposition à une vapeur de 115°C pendant 240 minutes est inférieur ou égal à 10 %.

5. Filtre de traitement du sang (10, 50) selon l'une quelconque des revendications 1 à 4, dans lequel le taux de changement de la chute de pression d'écoulement d'air avant et après exposition à la vapeur de 115° C pendant 240 minutes est inférieur ou égal à 2 %.

6. Procédé de traitement du sang comprenant l'étape de réalisation d'un traitement d'application d'une force centrifuge au filtre de traitement du sang (50) selon la revendication 2.

Figure 1

Figure 2

Figure 3

Figure 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2450431 A
- JP 60193468 A
- US 5580465 A
- JP 4134043 B
- JP 5710244 B

**Non-patent literature cited in the description**

- **SUEO KAWABATA.** The Standardization and Analysis of Texture Evaluation (second edition). the Textile Machinery Society of Japan, 10 July 1980
- Kagaku Binran (Handbook of Chemistry in English. Maruzen Publishing Co., Ltd, 1975, 164